# EUROPEAN PATENT APPLICATION

(11) **EP 1 618 842 A1**
(43) Date of publication of application: **25.01.2006**
(21) Application number: 05015819.5
(22) Date of filing: 20.07.2005
(51) Int. Cl.: A61B 5/053, A61B 5/0205

(54) **Medical information detection apparatus and health management system using the medical information detection apparatus**

(30) Priority: 20.07.2004 JP 2004211584; 20.07.2004 JP 2004211585; 20.07.2004 JP 2004211586; 20.07.2004 JP 2004211587; 20.07.2004 JP 2004211588
(71) Applicant: SHARP KABUSHIKI KAISHA, Osaka-shi, Osaka 545-8522 (JP)
(72) Inventor: Oishi, Yoshihiro, Ikoma-gun Nara 636-0144 (JP); Kotsuji, Hirotaka, Sakai-shi Osaka 591-8033 (JP); Higuchi, Shinichi, Osaka-shi Osaka 547-0026 (JP); Aoki, Fumihiko, Kashiba-shi Nara 639-0227 (JP)
(74) Representative: Müller - Hoffmann & Partner

(57) **Abstract**

A portable-type health management terminal 100 provided with a communication function and a management device 200 installed in a health management center are connected via a communication network N, wherein the health management terminal 100 is provided with a sensing unit that can sense medical information or an input device into which information about a state of health can be input, wherein the state of health is identified based on the information obtained from the sensing unit or the input device, and wherein these pieces of information are stored in a storage device, and are transmitted to the management device 200 through a communication module. The management device 200 stores the information that is received in a storage device 210, and manages the change in an individual's state of health based on the information that is stored. The sensing unit is provided with a pulse wave detection unit, constructed of a light emitting and receiving device or a pressure sensitive element, for sensing pulse waves, and an impedance detection unit constructed of a current application electrode and a voltage detection electrode.

## Description

### CROSS REFERENCE TO RELATED APPLICATIONS

This application claims priority on Patent Application No. 2004-211584, Patent Application No. 2004-211585, Patent Application No. 2004-211586, Patent Application No. 2004-211587 and Patent Application No. 2004-211588 filed in Japan on July 20, 2004, the entire contents of which are hereby incorporated by reference.

### BACKGROUND OF THE INVENTION

### 1. Field of the Invention

The present invention relates to medical information detection apparatuses that have functions for sensing medical information, and to health management systems using the medical information detection apparatuses.

### 2. Description of the Related Art

Daily health management is essential for the prevention of lifestyle diseases (preventing high blood pressure and obesity), and so from this viewpoint, blood pressure and body fat percentage should be measured on a regular basis. Thus, electric sphygmomanometers and body fat scales for households have come into general use in recent years.

As disclosed in JP 2002-51993, general sphygmomanometers up to now have been provided with a cuff. A person's blood pressure is measured by a method in which, firstly, the cuff is wrapped around the upper arm portion, or arm, for example, and is then inflated with air to stop the blood flow by compressing the arm, for example. After this, the pressure in the cuff is steadily decreased, and the blood pressure level at which blood starts flowing again is taken to be the systolic pressure. The pressure in the cuff is then reduced further, and the blood pressure level at which the sound of the blood flow ceases to be heard is taken to be the diastolic pressure. With such a conventional method for measuring blood pressure, there have been issues such as the bother of wrapping the cuff, and the distress caused by the air pressure in the cuff.

Also, as in JP 2001-070258A, for example, a sphygmomanometer has been proposed with a body fat percentage measuring instrument attached, however with this proposed sphygmomanometer, it is still necessary to attach a cuff to the arm.

As a technique for solving such issues, one, such as in JP 2000-107141A, has been disclosed in which pulse waves are measured at two or more positions on the body, wherein the time difference to the second pulse is determined with reference to the first pulse wave, and a blood pressure level is calculated from that value.

Furthermore, as another method for alleviating the subject's distress, there is also a measurement method for determining blood pressure wherein the pulse wave and the electrocardiac signals are measured simultaneously, and the fact that there is a correlation between the time difference between the pulse wave and the electrocardiac signals, and blood pressure level is utilized such as in JP H4-200439A, for example.

On the other hand, body fat scales for measuring body fat percentage, which has recently come to attention as an index for preventing lifestyle diseases, are provided with a plurality of electrodes that can externally contact the subject, such as in JP 2002-159461A, wherein the impedance of the subject's body can be measured through contact with these electrodes. A formula for calculating the body fat percentage from the measured impedance is also stored. By inputting data such as the subject's height, weight, sex and age, as their own personal physical data, and then measuring the impedance, the subject's body fat percentage may then be determined by calculations based on this physical data and the impedance of his body.

However, if a method for determining the blood pressure based on the time difference between the pulse wave and the electrocardiac signal is employed when measuring blood pressure and body fat percentage, then the electrode unit is used to measure the electrocardiac signal and it has been difficult to measure the blood pressure and body fat percentage at the same time. Thus, up to now, sphygmomanometers and body fat scales have been configured as individual apparatuses. Consequently, it has not only been necessary to measure blood pressure and body fat individually with separate apparatuses, wherein the measurement procedure is complex, but the time required for measurement is long. Furthermore, if the user desires to measure his blood pressure and body fat percentage while traveling, then it has been necessary to take both the sphygmomanometer and the body fat scales with them, and this has been considerably arduous.

From another point of view, in the "aging society" that is to come, there will be a need to transfer some work and services to robots. Thus, it will be necessary for people or living organisms to coexist with robots, and there is a need for sensors that can sense people's medical information. However, in current robots, this sensing function is substantially realized by sensors for distinguishing between people and objects by analyzing information obtained from imaging elements such as CCDs, or by sensors that differentiate between the two by sensing a state that is under pressure due to contact with a pressure sensor, for example. In other words, the sensors perform sensing using a visual function, or they make estimates from sensing using heat, sound, pressure and touch sensors, for example, rather than sensing a person's medical information directly.

Associated with such techniques, conventionally, in JP 2000-5317A for example, an apparatus has been proposed in which a device that is mounted on a person's body for directly sensing the person's medical information is linked to a pet-type robot.

The apparatus is provided with a detection unit for detecting a person's medical information, and the apparatus stores medical information detected in advance in a storage unit as comparative information. Then, by comparing medical information that is detected again by the detection unit while in a "condition control support mode" with the comparative information stored in the storage unit, the pet-type robot has a control configuration such that it takes independent action based on the comparative results. More specifically, this apparatus is formed so that it is easy for people to attach it to their body, in the manner of a wristwatch, spectacles, card or pedometer, and the apparatus is configured so as to control the pet-type robot. Thus, conventional pet-type robots are simply treated as objects to be controlled by the apparatus that detects the medical information, and the present situation is that a configuration in which sensors for directly sensing a person's medical information are provided on any of a robot's structural parts has not been proposed at the present time.

Moreover, conventionally, it has generally been the case that health management has been carried out based on tests performed in medical institutions and the like, but such a method cannot continuously capture variations in physical condition, and there is no information prior to or subsequent to the change in the subject's physical condition. Thus, there is no basic data available so it is difficult to give appropriate treatment, particularly when it is necessary to act during an emergency. Furthermore, there is also a necessity for continuous data in order to manage daily health with a health management system.

On the other hand, a health management system has been proposed, such as in JP H11-306468A and JP 2000-132621A, wherein a health management apparatus is attached to a toilet or bath or the like, and necessary information is obtained when the service user uses the bath or the toilet. However, in these conventional health management systems, there has been a problem in that medical information cannot be obtained unless the service user uses the toilet or bath, or the like, to which the health management apparatus has been attached.

Other examples of the conventional art have also been proposed, such as in JP H8-38435A in which a home-based health management system is provided such that medical information can be obtained rapidly from any desired location within the home by the service user carrying a mobile instrument that can sense medical information. In this conventional health management system, although medical information can be obtained at any time and at any location within the home, there is a problem that it is completely unusable when the subject goes out.

### SUMMARY OF THE INVENTION

The present invention has been accomplished with consideration of such facts, and it is an object thereof to provide a medical information detection apparatus that is capable of measuring systolic pressure, diastolic pressure, pulse rate and body fat percentage at the same time with a single measurement, and to provide a favorable health management service by acquiring such personal medical information at any time and in any place.

The medical information detection apparatus of the present invention is characterized in that movement of blood that is pumped from a heart is regarded as a pulse wave, and the medical information detection apparatus includes pulse wave detecting means constructed of a light emitting and receiving device, or a pressure-sensing element, for detecting the pulse wave, and impedance detecting means constructed of a current application electrode and a voltage detection electrode. By this configuration, it is possible to measure the blood pressure, pulse rate and body fat percentage by the pulse wave detecting means and impedance detecting means.

First, as described above, for blood pressure measurement, the blood flow is stopped by a pressure band known as a cuff, wherein the systolic pressure and diastolic pressure are determined by gradually lowering the pressure. It is known that there is a correlation between pulse wave propagation time, determined by the time difference of the pulse rate at two places using means for detecting pulse rate, and blood pressure level (see JP2000-107141A, for example).

Since there are variations between individuals between the correlation of the pulse wave propagation time and the blood pressure, it is necessary to measure the systolic pressure and diastolic pressure in advance. More specifically, a highly precise measurement can be obtained by determining the relationship formula (correction formula) between systolic pressure value and diastolic pressure value that have already been measured with another sphygmomanometer, and the actual systolic pressure value arid diastolic pressure value based on the pulse wave propagation time to correct the actual measured blood pressure level in accordance with that relationship formula.

In the present invention, the medical information detection apparatus then determines the pulse propagation time from the detection output of the pulse wave detecting means, and the systolic pressure, diastolic pressure and pulse rate at the actual time of measurement are measured based on the correlation of the pulse wave propagation time and blood pressure. Moreover, by determining the impedance value of a person's body by impedance detecting means constructed of a current application electrode and a voltage detection electrode, body fat percentage can also be calculated and so it is possible to measure the systolic pressure, diastolic pressure, pulse rate and body fat percentage at the same time in one measurement.

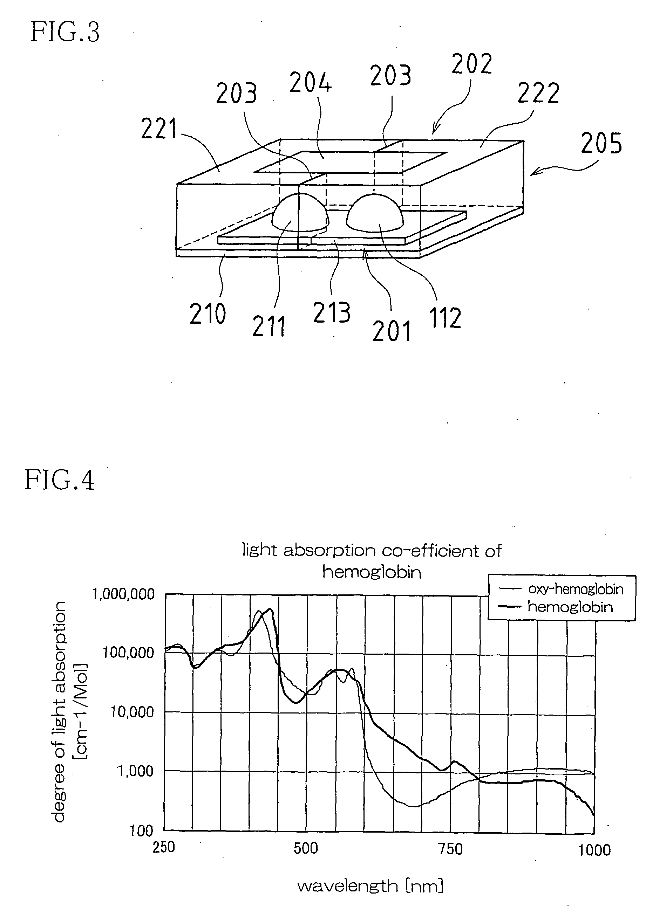

In order to sense the pulse rate in blood vessels, it is necessary to detect the movement of blood within the blood vessel. Thus, in an embodiment of the present invention, the medical information detection apparatus is configured to sense the movement of the blood by utilizing the light absorption spectrum characteristics of oxy-hemoglobin of red blood platelets contained in the blood (see FIG. 4), wherein when light of a specific wavelength is shone onto the blood, the amount of reflected light is reduced by absorption by the oxy-hemoglobin. The medical information detection apparatus is also configured to use two kinds of devices as the sensing means, namely a light emitting device and a light receiving device. As can be understood by the light absorption spectrum characteristics shown in FIG. 4, if the wavelength is selected simply on the degree of light absorption, then light in the vicinity of 400 nm is favorable, however with consideration to the scattering properties of light and to the confusion caused by moisture absorption, for example, it is preferable to select an appropriate wavelength in accordance with the application and purpose.

The light receiving device used in the present invention is a device for sensing light emitted from a light emitting device onto a person's finger, for example, that is then reflected by components in the blood, but if it can be expected to pickup light such as sunlight or light from fluorescent tubes, then a plurality of light receiving devices should be provided on the light emitting and receiving device.

Furthermore, when the light that is emitted from the light emitting device is only one wavelength, absorption of the light by oxy-hemoglobin in the blood may not be detected sufficiently, and so to increase the detection accuracy it is favorable to configure the light emitting and receiving device so as to detect the pulse wave using light of two or more different wavelengths.

Moreover, for the light emitting and receiving device used in the present invention, the wavelength components of the light emitting device, and the wavelength components of light such as sunlight and fluorescent light will be detected as described above, and so because the amount of oxy-hemoglobin is detected, provided that the device is a light receiving device, a configuration may be employed wherein a plurality of light emitting devices for emitting light of a plurality of wavelength components, and a plurality of light receiving devices for receiving light of a plurality of wavelength components are mounted in accordance with the situation in which they are used.

Furthermore, because the amount of oxy-hemoglobin in the blood is detected while detecting the pulse wave, it is preferable to use light emitting devices that emit light having a wavelength component of about 780 to 1000 nm, which is a region of near infrared light and infrared light, as the wavelength of the light emitted by the light emitting element, and the light sensitive wavelength of the light receiving element of the light emitting and receiving device used in the present invention. In terms of the light absorption characteristics of oxy-hemoglobin in the blood, utilizing the property that the absorption of light having a wavelength component of 360 to 660 nm is high, it is particularly preferable to use light emitting devices that emit light having that wavelength component (360 to 660 nm).

In the present invention, in order to detect the amount of oxy-hemoglobin by near infrared light and infrared light, the near infrared light and infrared light can be efficiently sensed provided that the medical information detection apparatus is configured so that the light emitting device and the light receiving device are sealed in resin that contains a component that cuts visible light.

Furthermore, in order to detect the amount of oxy-hemoglobin with near infrared light and infrared light, the near infrared light and the infrared light can be efficiently sensed when a filter for cutting visible light is arranged on the optical axis of the light emitting device and the light receiving device.

In the present invention, a light emitting and receiving device that is mounted with an IrDA (Infrared Data Association) device may be used as the light emitting and receiving device that constitutes the pulse wave detecting means.

In the present invention, it is necessary to press a part of a person's body with appropriate pressure when detecting the pulse wave, however detection may be difficult if the pressure that is used is greater than the necessary pressure. As one means for solving this problem, it is possible to give the example of a configuration in which a projecting portion is provided on the perimeter portion of the pulse wave detecting means. When such a projecting portion is provided, the light emitting and detecting device can make measurements with an appropriate pressure, even if the pressure that is applied is greater than necessary.

For the present invention, it is possible to configure the pulse wave detecting means for sensing the flow of blood, and the impedance detecting means for measuring a person's impedance in a single piece so as to use them as the equivalent of a single sensor. That is to say, in order to configure these to simultaneously measure blood pressure, pulse rate and body fat percentage, it is necessary to provide the pulse wave detecting means and the impedance detecting means as the sensing means that contacts a part of a person's body, such as a finger, in the same location. Thus it is necessary to compactly combine the pulse wave detecting means and the impedance detecting means. Here, since the method for sensing the medical information is an optical method for the pulse wave, and an impedance method for body fat percentage, these cannot be sensed by the same method. Consequently, in the present invention, the pulse wave detecting means and the impedance detecting means can be arranged in the same location by collecting the sensing means of varying methods into the same package and forming them as a single piece, as described above.

In the present invention, the pulse wave is measured at two locations, and the pulse wave propagation time that is calculated from these two pulse waves is utilized to calculate the blood pressure. In order to realize this, it is necessary to arrange first pulse wave detecting means and second pulse wave detecting means in at least two locations. When obtaining blood pressure using the two pulse wave detecting means, a pulse wave signal obtained from the first pulse wave detecting means and a pulse wave signal obtained from the second pulse wave detecting means may be sent to a blood pressure calculating unit to calculate the pulse wave propagation time. Next, processing to determine the actual systolic pressure value and diastolic pressure is performed by correcting the systolic pressure value and the diastolic pressure value is corrected in accordance with the systolic pressure and diastolic pressure already input, and the history of blood pressure measurements up to the present that are in the storage device, and calculating the systolic pressure and the diastolic pressure.

As a more specific configuration of the present invention, it is possible to give the configuration of, for example, pulse wave propagation time calculating means for calculating the pulse wave propagation time based on signals detected by the pulse wave detecting means, impedance calculating means for calculating the impedance of a living organism based on signals detected by the impedance detecting means, and storing means for storing the calculated results, wherein the pulse wave propagation time calculating means, the impedance calculating means and the storing means are formed as a single piece.

In the present invention, if a configuration is employed wherein the light emitting and receiving device is fixed to a holder, and the current application electrode and the voltage detection electrode are formed as a single piece with the holder, then it is possible to collect the pulse wave detecting means and the impedance detecting means, both of which have different sensing methods, into one.

In the present invention, if a configuration is employed wherein resin is filled between the light emitting and receiving device and the current application electrode and the voltage detection electrode, and wherein the light emitting and receiving device, and the current application electrode and the voltage detection electrode are formed as a single piece in the sealing resin, then it is possible to collect the pulse wave detecting means and the impedance detecting means, both of which have different sensing methods, into one.

In the present invention, if a configuration is employed wherein a front peripheral portion of the light emitting and receiving device is sealed with an electrically conductive resin, and the current application electrode and the voltage detection electrode are formed by the electrically conductive resin for sealing, then it is possible to collect the pulse wave detecting means and the impedance detecting means, both of which have different sensing methods, into one. As well, it becomes unnecessary to separately form the current application electrode and the voltage detection electrode.

A medical information detection apparatus of one embodiment of the present invention is provided with a configuration wherein a plurality of the pulse wave detecting means, a plurality of the impedance detecting means, blood pressure measuring means for receiving a pulse wave signal from the plurality of pulse wave detecting means and determining a blood pressure level from the time difference between the pulse waves detected at the two or more locations, and body fat percentage calculating means for calculating body fat percentage from impedance values between the plurality of impedance detecting means and physical data that is input in advance, are contained in a portable casing (a mobile medical information detection apparatus) .

With this embodiment, it is possible to receive a pulse wave signal obtained from a light emitting element of a single pulse wave sensing unit, and a pulse wave signal obtained from a light receiving element of another one pulse wave sensing unit, and the blood pressure level can be determined from the time difference between these pulse waves. On the other hand, the body fat percentage calculating means calculates the body fat percentage based on data such as the impedance values between the impedance sensing units, and the subject's weight, height age and sex. In this way, the blood pressure and the body fat can be measured at the same time by a compact (portable) medical information detection apparatus.

Furthermore, one of the pulse wave detecting means and one of the impedance detecting means are formed as a single piece so as to sense a single location on a body.

As a more specific applied embodiment of the portable medical information detection apparatus having such a configuration, there is a configuration wherein the medical information detection apparatus is incorporated with a flip-type mobile phone as a single piece, and blood pressure and body fat percentage are measured while the mobile phone is in a closed state. As another suitable aspect, there is a configuration wherein the medical information detection apparatus is incorporated with a flip-type mobile phone as a single piece, and blood pressure and body fat percentage are measured while the mobile phone is in an unfolded state. There is also a configuration wherein the medical information detection apparatus is incorporated with a non-folding-type mobile phone as a single piece to measure blood pressure and body fat percentage.

With this configuration, it is possible for the subject to not appear as though he is taking his blood pressure and body fat measurements, and rather appear as though he is operating a mobile phone, so as to measure blood pressure and body fat at any time and in any place without attracting attention.

If a display unit is provided for displaying the measured results of the blood pressure level and the body fat percentage, then the subject can easily and quickly confirm the measured results, and the usability of the portable medical information detection apparatus can be greatly enhanced.

Furthermore, by using an IrDA light emitting element and light receiving element as the pair of the light emitting element and the light receiving element, the configuration of the conventional communication function can be used as is while including a blood pressure measurement function.

In this case as well, it is possible to make a configuration wherein the medical information detection apparatus is incorporated with a flip-type mobile phone as a single piece, and blood pressure and body fat percentage are measured while the mobile phone is in a closed state. It is also possible to make a configuration wherein the medical information detection apparatus is incorporated with a flip-type mobile phone as a single piece, and blood pressure and body fat percentage are measured while the mobile phone is in an unfolded state. Furthermore, it is also possible to have a configuration wherein the medical information detection apparatus is incorporated with a non-folding-type mobile phone as a single piece to measure blood pressure and body fat percentage.

With these configurations as well, it is possible for subjects to not appear as though they are taking their blood pressure and body fat measurements, and rather appear as though they are operating a mobile phone, so as to measure blood pressure and body fat at any time and in any place without attracting attention.

By using the electrodes of the impedance detecting means as electrodes of a sensor for recognizing fingerprints, the configuration for a conventional fingerprint recognition function can be used as is, while including a body fat measurement function.

Furthermore, as a configuration wherein the above-mentioned portable medical information detection apparatus can communicate with an external device, it is possible to set a configuration wherein information of the blood pressure level and the body fat percentage, which are the measured results, can be transmitted to an external device by the IrDA device, and wherein physical data of the subject can be obtained from the external device.

Moreover, it is also possible to mount a blood sugar level sensor on the portable medical information detection apparatus. The blood sugar level sensor in this case may be one that measures blood sugar level by a chemical detection method, or may be one that measures blood sugar level by an optical detection method.

It is also possible to set a configuration to transmit the measured medical information to a medical institution, for example by a communication function such as the internet, and after a doctor has diagnosed the medical information, to receive the results of the diagnosis using the communication function, such as the internet.

Additionally, it is also possible to set a configuration to execute a piece of application software with an application software executing function, based on the measured medical information.

By providing an alarm function for notifying a time to measure blood pressure and body fat, it is also possible to measure blood pressure and body fat regularly.

Examples of devices that may incorporate the portable medical information detection apparatus, other than mobile phones, are given below. Firstly, there is a configuration for measuring medical information, incorporated in a personal computer. There is also a configuration for measuring the medical information, incorporated into a mouse for using the personal computer.

With the portable medical information detection apparatus of the present invention, the medical information can be measured at any time and in any place by providing the medical information measuring function in an information terminal device that is carried all the time. Furthermore, because the medical information detection apparatus can be mounted in a small space by forming the sensing unit in a single piece, the action of measuring the medical information can be made to appear as if a person is normally operating a mobile phone, and the measurements can be taken without attracting attention. Moreover, if a storage function is provided, then it is possible to store the daily measured results, and to confirm changes in the medical information. Furthermore, by fitting a transmission function it is possible to perform such actions as transmitting the measured results to an external device, tabulating and statistically analyzing the measured results, transmitting the measured results to a medical institution, getting a doctor's diagnosis, and making an appointment for a consultation. Additionally, by fitting an alarm function it is possible to take measurements every day at a pre-determined time without forgetting, and by providing an application software execution function, it is possible to run applications that utilize changes in the medical information, and to perform one's own health management at any time and in any place.

A medical information detection apparatus serving as one embodiment of the present invention is provided with a configuration wherein a plurality of pulse wave sensing units constituted by light emitting elements and light receiving elements, a plurality of impedance sensing units for measuring a body's impedance, blood pressure measuring means for receiving a pulse wave signal from the plurality of pulse wave sensing units and determining a blood pressure level from the time difference between the pulse waves detected at the two or more locations, and body fat percentage calculating means for calculating body fat percentage from impedance values between the plurality of the impedance sensing units and physical data that is input in advance, are incorporated in an interior component of a vehicle (vehicle-mounted medical information detection apparatus).

With this configuration, by the action of the driver, or passengers of the vehicle touching the medical information detection apparatus that is incorporated into the interior component of the vehicle, the blood pressure measuring means receives a pulse wave signal obtained from the light receiving element of a single pulse wave sensing unit, and a pulse wave signal obtained by the light receiving signal of another one pulse sensing unit, and the blood pressure level can be obtained from the time difference of these pulse waves. On the other hand, the body fat percentage calculating means calculates the body fat percentage based on the impedance value from between the impedance sensing units, and data such as the subject's weight, height, age and sex. In this way, it is possible to measure blood pressure and body fat percentage simultaneously by the medical information detection apparatus, even when riding in the vehicle.

More specifically, a steering wheel, shift lever and seat are examples of the interior component of the vehicle.

One of the pulse wave detecting means and one of the impedance detecting means are formed as a single piece so as to sense a single location on a body.

By using an IrDA light emitting element and light receiving element also as the pair of the light emitting element and the light receiving element, the configuration of the conventional communication function can be used as is while including a blood pressure measurement function.

In this case, the medical information detection apparatus may be configured so that blood pressure level information and body fat percentage information, which are the measured results, can be transmitted to the external device by the IrDA device, or such that the physical data of the subject can be obtained from the external device. With this, it is possible to store the blood pressure level information and the body fat percentage information in the external device, or by calling up the physical data of the subject that is stored in the external device, it is possible to eliminate the work required to input the physical data with every measurement.

Moreover, it is also possible to include a blood sugar level sensor in the vehicle-mounted medical information detection apparatus. In this case, the blood sugar level sensor may be a sensor in which blood sugar level is measured by a chemical detection method, or may be a sensor in which blood sugar level is measured by an optical detection method.

An operation that judges whether or not the subject is in a condition to be able to drive, based on the measured results, and that notifies that judgment to the subject; an operation that adjusts a set airflow or set temperature of an airconditioning apparatus of the vehicle based on the measured results, and an operation that adjusts the volume of an acoustic device of the vehicle based on the measured results are examples of operations that reflect the measured results described above.

With the vehicle-mounted medical information detection apparatus of the present invention, blood pressure and body fat percentage can be measured while driving the vehicle, thus improving its usefulness. Furthermore, it is possible to provide a pleasant environment inside the cabin by adjusting the state of the airconditioning, and the acoustic state, in accordance with the measured results.

Moreover, an embodiment of the medical information detection apparatus (a robot-type medical information detection apparatus) of the present invention contains a sensor provided with the pulse wave detecting means and the impedance detecting means in any structural part of a robot that has a numerical input function, a display function, a transmission and receiving function, a storage function, an alarm function, an application software execution function, a calculation function and a control function as basic functions. The sensor is provided on a part of the robot that touches a living organism. Furthermore, the medical information sensed by the sensor is at least one or more of an electrocardiac signal, blood pressure, pulse rate, pulse wave, body temperature, body fat, bone density, blood oxygen concentration and blood sugar level.

In the robot-type medical information detection apparatus, when using an optical method for measuring blood pressure and pulse rate, it is possible to determine the pulse rate by sensing, with the sensor, utilizing the light absorption characteristics of the oxy-hemoglobin, which is the hemoglobin in the blood which has the greater amount of oxygen.

If the sensor is constructed of a pressure sensitive element, then the pulse rate can be determined by sensing pressure fluctuations of the blood vessels by the pressure sensitive element.

Furthermore, in addition to the methods described previously, blood pressure can be determined by calculation from the time difference between electrocardiac signals and pulse waves. A technique for determining blood pressure level by calculation from the time difference between electrocardiac signals and pulse waves is disclosed in Japanese Patent No. 3028601, for example.

If the pulse wave is measured by an optical method, then using a sensor constructed of a combination of the light emitting element and the light receiving element, it is possible to measure the pulse wave by utilizing the light absorption characteristics of the oxy-hemoglobin, which is the hemoglobin in the blood which has the greater amount of oxygen, as described above. Furthermore, if the pulse wave is measured by a pressure method, then the pressure sensitive elements obtain the fluctuations in the pressure in the blood vessel, and it is possible to measure the pulse wave from the changes in the way the pressure fluctuates.

For the blood oxygen concentration (SpO2) as well, if the medical information detection apparatus measures the pulse wave by an optical method, then it is possible to measure the degree of light absorption of oxy-hemoglobin in the blood by using a plurality of wavelengths of light using a sensor constructed of a combination of the light emitting element and the light receiving element.

It is also possible to determine body fat or bone density from calculations by passing an electric current between two measurement regions, and measuring the impedance of the living tissue by the voltage between those two points. Moreover, the electrocardiac signals can be determined by measuring with an impedance method between two points on the body. Information regarding measurement with this impedance method is disclosed in [Title: "*Hajimete shindenzu wo manabu katagata e"* (Electrocardiographs for beginners), Author: Touki Moritani, Publisher: Houmeido Shoten].

It is possible to determine the body temperature by using a pyroelectric sensor as the sensor, by measuring inside the ear with the pyroelectric sensor. Moreover, if blood sugar level is measured by an optical method, then it is possible to use a sensor constructed of a plurality of near infrared light sources and light receiving elements to contactlessly measure the blood sugar level.

The robot-type medical information detection apparatus of the present invention may be provided with a network connection function. By communicating with other devices using the network connection function, it is possible to obtain necessary information from the other devices.

The robot-type medical information detection apparatus of the present invention is configured such that a sensor that has a function that can sense medical information is provided on any structural part of the robot, and thus the robot can be utilized not just as a conventional industrial robot, but also as a care robot and a medical robot. Moreover, it is also possible to utilize the robot as a pet-type robot or as a recreational robot.

One embodiment of a health management system of the present invention includes a portable-type health management terminal provided with a communication function, and a management device in a health management center that can be connected via a communication network, wherein the health management terminal includes at least either one of sensing means constructed of a medical information detection apparatus according to any one of claims 1 to 71 that can sense medical information; or inputting means into which a health state can be input, judging means for judging the health state based on the information obtained by these means, storing means for storing the information, and transmitting means for transmitting the information and the information of the judgment result via the communication network, and wherein the management device includes storing means for storing, in a storage device, the information that is transmitted, and managing means for managing the transition of an individual's health state based on the information stored in the storage device.

Thus, by always carrying the health management terminal provided with the communication function when going out, the service user, who is the user, can measure his own medical information at any time, and at any place, and can transmit that information to the management device. Thus, the management device is able to grasp continuous fluctuation in the user's physical condition, the management device can also judge the presence of abnormalities, or the degree of urgency, based on changes in physical condition, and can provide an appropriate service to the service user (the user).

Here, a mobile phone is suitable as the health management terminal, and it is preferable that the sensing means is disposed in the vicinity of a location to which a finger will naturally come when the mobile phone is being used. Thus, when the service user uses the mobile phone by making or answering a call, the medical information can be sensed naturally.

In this case, the information that is sensed by the sensing means may be the pulse wave, or a living organism's impedance. In other words, the sensing means may be provided with a light emitting and detecting means for pulse wave detection, and a current application electrode and a voltage detection electrode for detecting impedance. By using such a sensing means, it is preferable that the medical information collected by the health management terminal is at least any one of body temperature, blood pressure, pulse rate, pulse wave, heart rate, body weight, body fat, amount of internal organ fat, bone density, skin moisture, skin oil, blood oxygen concentration, blood sugar level, blood composition, cholesterol level, uric acid level, brain waves, stool amount and stool composition.

Furthermore, with the health management system of the present invention, the health management terminal is connected by the communication network to a terminal of a facility that can provide the necessary service to the user (the service user) who is carrying the health management terminal, and the management device transmits information regarding the terminal of the facility and provision of the service to the service user, based on the results of the identification of the state of his health.

The health management terminal may be configured so as to transmit data that includes information such as health insurance number, consultation ticket, ID card or drug history.

Thus, by connecting the management device to a terminal of facilities such as a medical institution or a welfare institution, if information that is transmitted from the health management terminal of the service user, for example, indicates a degree of urgency, then the management device can provide an appropriate service to the service user, such as making an appointment for a consultation to the terminal of a predetermined medical institution, based on information such as a health insurance number or consultation ticket (certificate) that has been received. That is to say, it is possible to construct a system that is useful for managing the health of a service user as a system for providing life saving treatment during an emergency, and health support at other times.

Here, the management device further comprises judging means for comparatively calculating the data that is transmitted from the health management terminal with the past data stored on the storage device, and judging the transition in the state of health of the service user. By providing such judging means, it is possible to accurately determine the state of health of the service user.

### BRIEF DESCRIPTION OF DRAWINGS

FIG. 1 is a perspective view schematically showing a first embodiment of a medical information detection apparatus of the present invention.

FIG. 2 is a perspective view schematically showing a second embodiment of the medical information detection apparatus of the present invention.

FIG. 3 is a perspective view schematically showing a third embodiment of the medical information detection apparatus of the present invention.

FIG. 4 is a graph showing the light absorbing characteristics of oxy-hemoglobin.

FIG. 5 is a block diagram showing a system configuration of a fourth embodiment of the medical information detection apparatus of the present invention.

FIG. 6 is a measurement block diagram showing a basic system structure for measuring blood pressure and body fat.

FIG. 7A is front view of a sensing unit suitable for the medical information detection apparatus of the present invention, and FIG. 7B is a lateral view.

FIG. 8 is a view showing how measurements are taken in a fifth embodiment.

FIG. 9 is a view showing a front side of a mobile phone, while in the open state, according to a sixth embodiment.

FIG. 10 is a view showing a back side of a mobile phone, while in the open state, according to a seventh embodiment.

FIG. 11 is a view showing a front side of a mobile phone, according to an eighth embodiment.

FIG. 12 is a view showing a back side of a mobile phone, according to a ninth embodiment.

FIG. 13 is a view corresponding to FIG. 8 according to a tenth embodiment.

FIG. 14 is a view corresponding to FIG. 9 according to an eleventh embodiment.

FIG. 15 is a view corresponding to FIG. 11 according to a twelfth embodiment.

FIG. 16 is a view corresponding to FIG. 9 according to a thirteenth embodiment.

FIG. 17 is a view showing a mobile phone and an external apparatus according to a fourteenth embodiment.

FIG. 18 is a view corresponding to FIG. 9 according to a fifteenth embodiment.

FIG. 19 is a view corresponding to FIG. 9 according to a sixteenth embodiment.

FIG. 20 is a perspective view of a personal computer according to a seventeenth embodiment.

FIG. 21 is a perspective view of a mouse according to an eighteenth embodiment.

FIG. 22 is a view showing a steering wheel and an external apparatus according to a nineteenth embodiment.

FIG. 23 is a view showing a steering wheel, a shift lever, and an external device according to a twentieth embodiment.

FIG. 24 is a view showing a steering wheel, a gear stick and an external device according to a twenty first embodiment.

FIG. 25 is a view showing a seat according to a twenty second embodiment.

FIG. 26 is a plan overview showing a constituent example of a sensor unit for sensing medical information according to the present invention.

FIG. 27 is an explanatory diagram showing how the sensor unit is attached to finger tips of a robot.

FIG. 28 is a block diagram showing a system configuration of the robot on which the sensor unit is mounted, according to the present invention.

FIG. 29 is a block diagram showing an overall configuration of the health management system of an embodiment of the present invention.

FIG. 30 is an explanatory diagram showing an example of an arrangement configuration when two sensor units are disposed on a flip-type mobile phone.

FIG. 31 is an explanatory diagram showing another example of an arrangement configuration when two sensor units are disposed on a flip-type mobile phone.

FIG. 32 is an explanatory diagram showing still another example of an arrangement configuration when two sensor units are disposed on a flip-type mobile phone.

FIG. 33 is a flow chart showing process operations of a health management terminal.

FIG. 34 is a flow chart showing process operations of a management device.

FIG. 35 is a flow chart showing process operations of the management device.

### DESCRIPTION OF THE PREFERRED EMBODIMENTS

Embodiments of the medical information detection apparatus of the present inventions are described below with reference to the drawings.

### First Embodiment

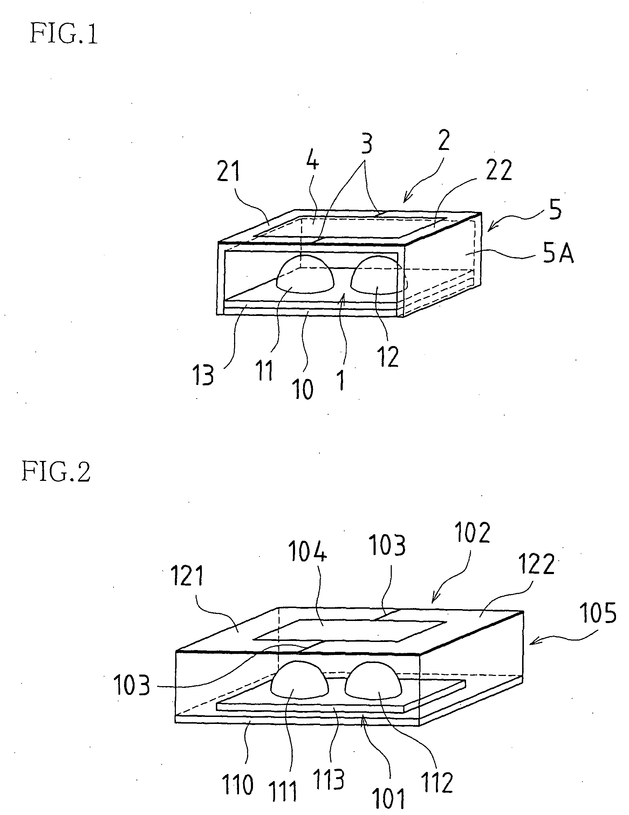

FIG. 1 is a perspective view schematically showing a first embodiment of the medical information detection apparatus of the present invention.

The medical information detection apparatus of the first embodiment has a light emitting and receiving device 1 as pulse wave detecting means, impedance detecting means 2, separators 3, a visible-light cutting filter 4 and a hollow rectangular holder 5.

The light emitting and receiving device 1 is disposed within the holder 5. The light emitting and receiving device 1 has a light emitting device 11 and a light receiving device 12 mounted on a substrate 10, and the light emitting device 11 and the light receiving device 12 are sealed by a sealing resin 13.

The impedance detecting means 2 has a current application electrode 21, and a voltage detection electrode 22. The current application electrode 21 and the voltage detection electrode 22 are formed along peripheral portions of the front face of the rectangular holder 5. The current application electrode 21 and the voltage detection electrode 22 are separated by the separators 3. The visible-light cutting filter 4 is then disposed in a position at the front of the light emitting and receiving device 1 (on the optical axis of the light emitting device 11 and the light receiving device 12), in a central portion between the current application electrode 21 and the voltage detection electrode 22.

It should be noted that with consideration to the light absorption characteristics of oxy-hemoglobin in blood, it is preferable to use a light emitting device in which the wavelength of the emitted light is in the wavelength region of 360 to 660 nm as the light emitting device 11 of the light emitting and receiving device 1. In a similar manner for the light receiving device 12, it is preferable to use a light receiving device which is sensitive to receiving light in a wavelength band of 360 to 660 nm.

With the medical information detection apparatus in this example, the light emitting and receiving device (pulse wave detecting means) 1 for sensing the flow of blood and the impedance detecting means 2 for detecting the impedance of a person's body are formed in one piece, thus the systolic and diastolic pressures, the pulse rate, and the body fat percentage can be measured simultaneously in a single measurement. Moreover, the medical information detection apparatus of the present example is structured such that a protruding portion 5A is formed on the peripheral portion of the front side of the light emitting and receiving unit 1 by walls of the holder 5, and thus even if a person presses the front face of the medical information detection apparatus with pressure greater than is required when detecting the pulse wave, the pulse wave is always sensed at an appropriate pressure because the pressing force is regulated by the protruding portion 5A on the peripheral portion of the light emitting and receiving device 1.

It should be noted that if a visible-light cutting resin is used as the sealing resin 13 of the light emitting and receiving device 1 in the medical information detection apparatus of the present example, then there is no particular need to dispose the visible-light cutting filter 4 in the center portion between the current application electrode 21 and the voltage detection electrode 22, and the front of the light emitting and receiving device 1 may be covered by a transparent cover, for example.

### Second Embodiment

FIG. 2 is a perspective view schematically showing a second embodiment of the medical information detection apparatus of the present invention.

The medical information detection apparatus of the second embodiment has a light emitting and receiving device 101 as the pulse wave detecting means, impedance detecting means 102, separators 103 and a visible-light cutting filter 104, wherein the light emitting and receiving means 101 and the impedance detecting means 103 are formed as a single piece in a piece of sealing resin 105.

The light emitting and receiving device 101 includes a light emitting device 111 and a light receiving device 112 mounted on a substrate 110, and the light emitting device 111 and the light receiving device 112 are sealed in a sealing resin (a primary mold resin) 113.

The impedance detecting means 102 has a current application electrode 121, and a voltage detection electrode 122. The current application electrode 121 and the voltage detection electrode 122 are formed along peripheral portions of the front face of the sealing resin 105 (the single piece resin), serving as a secondary mold resin. The current application electrode 121 and the voltage detection electrode 122 are separated by the separators 103. A visible-light cutting filter 104 is disposed in a position at the front of the light emitting and receiving device 101 (on the optical axis of the light emitting device 111 and the light receiving device 112), in a central portion between the current application electrode 121 and the voltage detection electrode 122.

It should be noted that with consideration to the light absorption characteristics of oxy-hemoglobin within the blood, it is preferable to use a light emitting device in which the wavelength of the emitted light is in the wavelength region of 360 to 660 nm as the light emitting device 111 of the light emitting and receiving device 101. In a similar manner, it is preferable to use a light receiving device which is sensitive to receiving light in a wavelength band of 360 to 660 nm as the light receiving device 112.

The medical information detection apparatus having the structure shown in FIG. 2 can be obtained by arranging the visible-light cutting filter 104 in the center portion between the current application electrode 121 and the pressure detecting electrode 122 of the impedance detecting means 102, and by forming the light emitting and receiving device 101 and the impedance detecting means 102 into a single piece as the pulse wave detecting means by filling the sealing resin 105 between the light emitting and receiving device 101, and the current application electrode 121 and the voltage detection electrode 122, while the current application electrode 121 and the voltage detection electrode 122 are installed, such that the visible-light cutting filter 104 is positioned at the front of the light emitting and receiving device 101 (on the optical axis of the light emitting device 111 and the light receiving device 112).

With the medical information detection apparatus in this example, the light emitting and receiving device (the pulse wave detecting means) 101 for sensing the flow of blood, and the impedance detecting means 102 for detecting the impedance of a person's body are formed in one piece, thus the systolic and diastolic pressures, the pulse rate and the body fat percentage can be measured simultaneously in a single measurement.

It should be noted that in the medical information detection apparatus of the present example, if a visible-light cutting resin is used as the sealing resin 105 for the secondary mold, then there is no particular need to dispose the visible-light cutting filter 104 in the center portion between the current application electrode 121 and the voltage detection electrode 122, and the front of the light emitting and receiving device 101 may be covered by a transparent cover, for example.

### Third Embodiment

FIG. 3 is a perspective view schematically showing a third embodiment of the medical information detection apparatus of the present invention.

The medical information detection apparatus of the third embodiment has a light emitting and receiving device 201 as pulse wave detecting means, impedance detecting means 202, separators 203 and a visible-light cutting filter 204.

The light emitting and receiving means 201 has a light emitting device 211 and a light receiving device 212 that are mounted on a substrate 210, and the light emitting means 211 and the light receiving means 212 are sealed in a piece of sealing resin (primary mold resin) 213.

It should be noted that with consideration to the light absorption characteristics of oxy-hemoglobin in blood, it is preferable to use a light emitting device in which the wavelength of the emitted light is in the wavelength region of 360 to 660 nm as the light emitting device 211 of the light emitting and receiving device 201. In a similar manner, it is preferable to use a light receiving device which is sensitive to receiving light in a wavelength band of 360 to 660 nm as the light receiving device 212.

In the medical information detection apparatus of the present example, the space on the front side of the light emitting and receiving device 201 is resin sealed (secondary molded) in an electrically conductive resin 205. Furthermore, the electrically conductive resin 205 is divided into two symmetrical shapes by the separators 203, and the medical information detection apparatus of the present invention is characterized in that the current application electrode 221 and the voltage detection electrode 222 that constitute the impedance detecting means 202 are formed in the divided conductive resin 205, and in that the visible-light cutting filter 204 is arranged in a central portion between the current application electrode 221 and the voltage detection electrode 222 that are made from the conductive resin 205, in a position at the front of the light emitting and receiving device 201 (on the optical axis of the light emitting device 211 and the light receiving device 212).

The above-noted medical information detection apparatus having the structure shown in FIG. 3 can be obtained by a technique wherein the visible-light cutting filter 204 is disposed at the front of the light emitting and the receiving device 201 (on the optical axis of the light emitting device 211 and the light receiving device 212), and wherein the light emitting and receiving device 201 and the visible-light cutting filter 204 are formed as a single piece by filling the conductive resin 205 into the space on the front side of the light emitting and receiving device 201 while the separators 203 are disposed in a predetermined position (the position dividing the current application electrode 221 and the voltage detecting electrode 222) on the front side of the light emitting and receiving device 201, while at the same time forming the current application electrode 221 and the voltage detection electrode 222 that constitute the impedance detecting means 202.

With the medical information detection apparatus of the present example, the current application electrode 221 and the voltage detection electrode are formed by filling the conductive resin 205 into the space on the front side of the light emitting and receiving device (the pulse wave detecting means) 201 for sensing the blood flow, thus the light emitting and receiving device 201 and the impedance detecting means 202 for detecting the body's impedance can be formed into a single piece, and systolic pressure, diastolic pressure and pulse, and the body fat percentage can be measured simultaneously in a single measurement. Moreover, since the current application electrode 221 and the voltage detection electrode are formed by the filling of the electrically conductive resin, there is no necessity to form the impedance detecting means separately, and it is possible to achieve a reduction in the number of components.

It should be noted that in the medical information detection apparatus of the present example, if a visible-light cutting resin is used as the conductive resin 205 that is used for the secondary mold, then there is no particular need to dispose the visible-light cutting filter 204 in the center portion between the current application electrode 221 and the voltage detection electrode 222, and the front of the light emitting and receiving device 201 may be covered by a transparent cover, for example.

### Fourth Embodiment

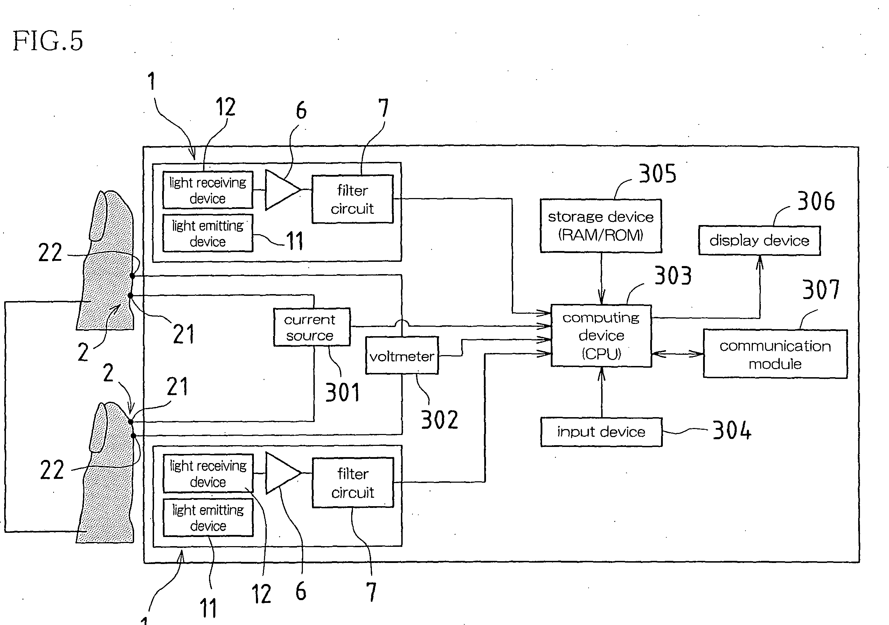

FIG. 5 is a block diagram showing the system configuration of the medical information detection apparatus of a fourth embodiment.

First, in order to measure blood pressure, it is necessary to detect the pulse wave at a plurality of locations (two locations in the fourth embodiment). Thus in the fourth embodiment, using two of the medical information detection apparatuses shown in FIG. 1, the light emitting and receiving devices (pulse wave detecting means) 1 are attached to a part (a sensing part) of a finger tip, and the impedance detecting units 2 constructed of the current application electrode 21 and the voltage detecting electrode 22 for measuring the body's impedance are attached to another part of the finger tip. The output of the light receiving devices 12 of the light emitting and receiving devices 1 attached in this way pass through amps 6 and filter circuits 7, after which they are received by a computing unit (CPU)303 that will be described later.

Furthermore, in the fourth embodiment, a current source 301 for applying an electric current to the current application electrodes 21, a voltmeter 302 for measuring the voltage between the two voltage detecting electrodes 22, the computing unit 303, an input device 304, a storage device (RAM/ROM) 305, a display device 306 and a communication module 307, and the like are provided as a measurement system.

The measurement process of the fourth embodiment is described next.

First, in the fourth embodiment, information such as the systolic pressure and diastolic pressure values, which have been measured by another sphygmomanometer, age, height, weight and sex are input by the input device 304 and are stored in the storage device 305. The two light emitting and receiving devices 1 and the two impedance detecting means 2 are then activated while attached to a left and a right finger respectively, as shown in FIG. 5, and the measurement is performed by applying a current to the current application electrodes 21 from the current source 301. Pulse wave signals from the two light receiving devices 12, a voltage signal measured in the voltmeter 302, and an applied current value from the current source 301 are input into the computing unit 303 in accordance with the measurement. As well as determining the pulse wave propagation time from the two pulse wave signals that were received, the computing unit 303 determines the body's impedance value based on the signal from the voltmeter 302 and the current source 301, and determines the systolic pressure, diastolic pressure, pulse rate and body fat percentage based on the pulse wave propagation time and the impedance value, and the previously entered values such as systolic pressure and diastolic pressure levels, and age, height, weight and sex. These calculated results are displayed on the display device 306, and are stored in the storage device 305. The results of the calculation of systolic pressure, diastolic pressure, pulse rate and body fat percentage may also be transmitted to and received from another external device using the communication module 307.

Describing the method used for computing blood pressure and body fat percentage in simple terms, it should be noted that in the fourth embodiment, blood pressure is determined by the relationship between the systolic pressure value and diastolic pressure levels measured by another sphygmomanometer (the values that were input previously), and the actual values of systolic pressure and diastolic pressure based on the pulse wave propagation time, and high measurement accuracy may be achieved by correcting the measured blood pressure levels in accordance with that relationship. Furthermore, for body fat percentage, the body fat percentage of the subject can be calculated based on a predetermined formula using the personal information such as age, height, weight and sex that is input in the input device 304, and the measured values of impedance.

Cases in which the medical information detection apparatus is constituted by employing mobile phones, laptop personal computers, and mouses that are connected to personal computers as mobile information terminal devices (referred to below as "portable-type health management devices"), are described next as embodiments of the medical information detection apparatus of the present invention.

### Basic System Structure of Portable-Type Health Management Device

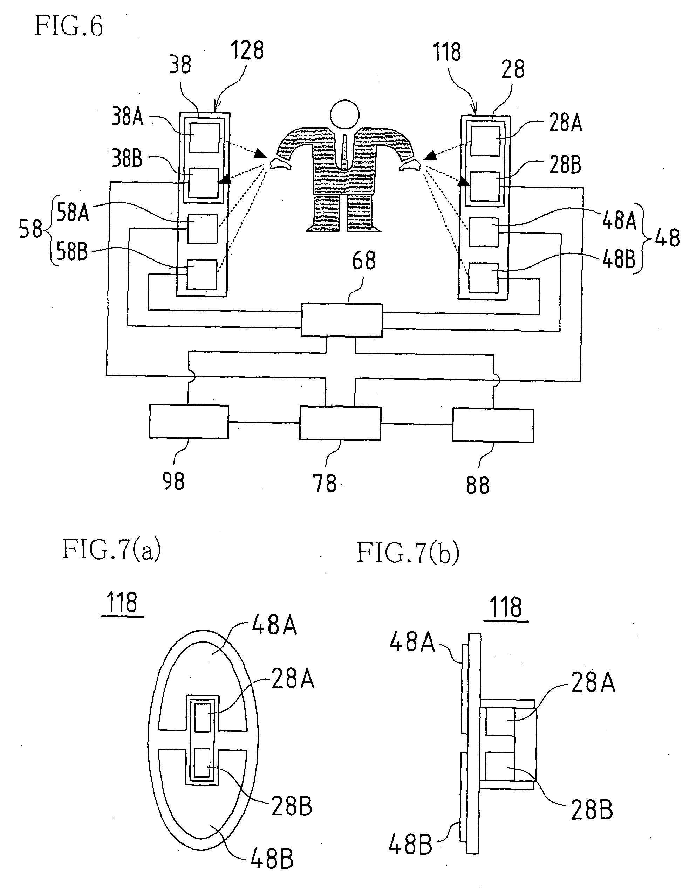

Before describing a specific structure of a portable-type health management device (a structure that is incorporated as a single piece into a mobile phone, for example), the basic system structure for carrying out blood pressure and body fat percentage measurements will be described using the measurement block diagram in FIG. 6.

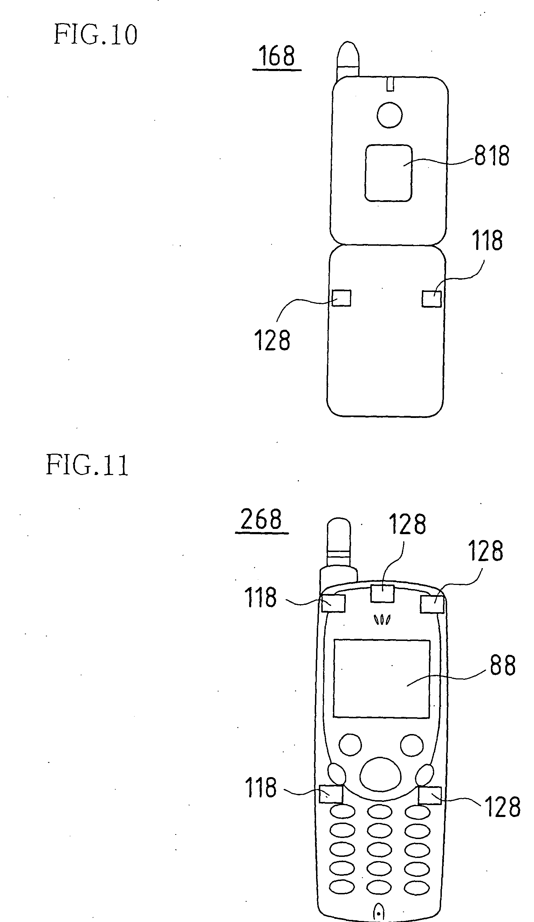

The present portable-type health management device as shown in FIG. 11 is provided with a first pulse sensing unit 28 to measure a pulse from the right hand, and a second pulse sensing unit 38 for measuring a pulse from the left hand.

While the first pulse wave sensing unit 28 is provided with a first light emitting element 28A and a first light receiving element 28B, the second pulse wave sensing unit 38 is provided with a second light emitting element 38A and a second light receiving element 38B. A blood pressure level measuring unit 78 is also provided for receiving the pulse wave signals obtained by the first light receiving element 28B and the second light receiving element 38B in FIG. 6.

With this configuration, the pulse wave signal of the left hand obtained by the first light receiving element 28B and the pulse wave signal of the right hand obtained by the second light receiving element 38B are transmitted to the blood pressure level measuring unit 78, wherein the blood pressure level is measured by the blood pressure level measuring unit 78. That is to say, it is possible to know the blood pressure level having a correlation with the pulse wave propagation time by determining the pulse propagation time that is determined by the time difference between the pulse wave (pulse rate) at the two regions. It should be noted that in order to sense the pulse rate in the blood vessel, it is necessary to detect the movement of the blood within the blood vessel. For example, the blood flow may be detected by using the characteristic light absorption spectrum of oxy-hemoglobin in red blood platelets in the blood, characterized in that when light of a certain wavelength is irradiated, the amount of light that is reflected is reduced by oxy-hemoglobin absorption.

Furthermore, the present portable-type health management device is provided with a first impedance sensing unit 48, and a second impedance sensing unit 58. The first impedance sensing unit 48 contacts the left hand, and is provided with a current application electrode 48A and a voltage detection electrode 48B. On the other hand, the second impedance sensing unit 58 contacts the right hand, and is provided with a current application electrode 58A and a voltage detection electrode 58B in a similar manner as the first impedance unit 48. Moreover, a numerical input unit 98 is provided that is capable of entering data such as the subject's weight, height, age and sex.

With this configuration, the body's impedance values measured by the impedance sensing unit 48 and 58, and the data, such as the subject's weight, height, age and sex that are input from the numerical input unit 98 are transmitted to a body fat percentage measuring unit 68, and the portable-type health management device is configured such that the body fat percentage is calculated in the body fat percentage measuring unit 68.

Blood pressure levels and information on body fat percentages that are obtained in this way are transmitted to the display unit 88 and are displayed on the display unit 88 such that the subject can visually confirm them.

Here, 780 to 1000 nm, which is the near infrared region, is used as the wavelength of the emitted light of the first and second light emitting elements 28A and 38A of the first and second pulse sensing units 28 and 38, and similarly, 780 nm to 1000 nm, which is the near infrared region, is used in the same way as the sensitive wavelength of the first and second light receiving elements 28B and 38B.

Furthermore, a wavelength of 360 to 660 nm may also be substituted and used as the wavelength of the emitted light of the first and second light emitting elements 28A and 38A of the first and second pulse wave sensing units 28 and 38, and a wavelength of 360 to 660 nm may be used as the wavelength sensed by the first and second light receiving elements 28B and 38B. This utilizes the fact that due to the optical absorption characteristics of oxy-hemoglobin in the blood, there is high absorption of light having wavelength components of 360 to 660 nm.

### Description of the Structure of the Sensing Portion

The structure of the pulse wave sensing units 28 and 38 and the impedance sensing units 48 and 58 are described in greater detail next. The first pulse sensing unit 28 and the first impedance sensing unit 48 for the left hand are configured in a single piece as a first sensing unit 118, and in a similar manner, the second pulse sensing unit 38 and the second impedance sensing unit 58 are configured in a single piece as a second sensing unit 128. Both the sensing units 118 and 128 have the same configuration. Thus, the first sensing unit 118 (left hand) will be described here as a representative example.

FIG. 7A is a front view of the first sensing unit 118, and FIG. 7B is a lateral view of the same. As shown in these diagrams, the first pulse sensing unit 28 and the first impedance sensing unit 48 are formed as a single piece to configure the first sensing unit 118, and the first sensing unit 118 is structured so that it can sense the pulse wave and the impedance of a part of the body (such as the finger tip) at the same location. More specifically, the front of the first sensing unit 118 is substantially oval shaped, and the first light emitting element 28A and the first light receiving element 28B are contained in a depressed portion formed in its center. The current application electrode 48A and the voltage detection electrode 48B are also provided on respective surfaces of the first sensing unit 118. It should be noted that the second sensing unit 128 also has the same configuration.

### How Sensing Units are Incorporated in a Mobile Phone

Next is a description of the configuration and measurement operation of a portable-type health management device when a device that is provided with the above-noted basic system structure is incorporated in a mobile phone.

### Fifth Embodiment

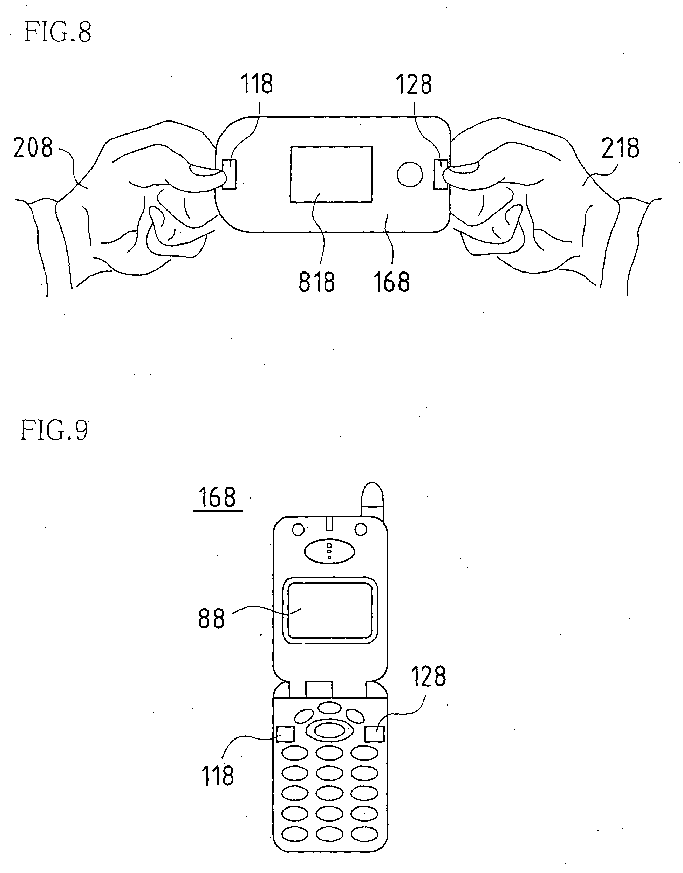

FIG. 8 shows a measurement operation in a case in which a portable-type health management device is incorporated as a single piece with a flip-type mobile phone 168.

As shown in FIG. 8, the sensing units 118 and 128 are provided respectively on both ends, in the longitudinal direction, of the mobile phone 168 when it is in a closed state. When measuring blood pressure and body fat percentage, the thumb of a left hand 208 contacts the first sensing unit 118 and the thumb of a right hand 218 contacts the second sensing unit 128 while the mobile phone 168 is closed. Thus, the pulse wave signal of the left thumb obtained by the first light receiving element 28B of the first sensing unit 118, and the pulse wave signal of the right thumb obtained by the second light receiving element 38B of the second sensing unit 128 are transmitted to the blood pressure level measuring unit 78, wherein the blood pressure level is measured. Also, the body's impedance value measured between the first impedance sensing unit 48 of the first sensing unit 118 and the second impedance sensing unit 58 of the second sensing unit 128, and data such as the subject's weight, height, age and sex that was input from the numerical input unit 98, are transmitted to the body fat percentage measuring unit 68, wherein the body fat percentage is calculated by the body fat percentage measuring unit 68. The blood pressure and body fat percentage that are determined in this way are displayed on a display screen 818 of the mobile phone (displayed on a sub screen when using a flip-type mobile phone 168).

### Sixth Embodiment

The present embodiment is one in which the portable-type health management device is incorporated as a single piece in a flip-type mobile phone 168 such that the blood pressure levels and the body fat percentage are measured while the mobile phone 168 is in an unfolded (open) state. In the present embodiment, the basic system structure for measuring the blood pressure and the body fat percentage, and the structure of the sensing units 118 and 128 is the same as described above, and thus their description is hereby omitted.

FIG. 9 shows the front side of the mobile phone 168 according to the present embodiment while in the open state. The first sensing unit 118 is disposed in a position in which the thumb of the left hand will be naturally placed when the mobile phone is held by a person with both hands, and the second sensing unit 128 is disposed in a position in which the thumb of the right hand will be naturally placed when the mobile phone is held by a person with both hands.

Thus, when measuring blood pressure and body fat percentage, the thumb of the left hand contacts the first sensing unit 118, and the thumb of the right hand contacts the second sensing unit 128 while the mobile phone is open. Thus, the pulse wave signal of the left thumb obtained by the first light receiving element 28B of the first sensing unit 128, and the pulse wave signal of the right thumb obtained by the second light receiving element 38B of the second sensing unit 128 are transmitted to the blood pressure level measuring unit 78, wherein the blood pressure level is measured. Also, the body's impedance value measured between the first impedance sensing unit 48 of the first sensing unit 118 and the second impedance sensing unit 58 of the second sensing unit 128, and data such as the subject's weight, height, age and sex that was input from the numerical input unit 98, are transmitted to the body fat percentage measuring unit 68, wherein the body fat percentage is calculated at the body fat percentage measuring unit 68. The blood pressure and body fat percentage that are determined in this way are displayed on a display screen (main screen) 88 of the mobile phone.

### Seventh Embodiment

The present embodiment is a modified example of the embodiment in which the portable-type health management device is incorporated as a single piece with the flip-type mobile phone 168 such that the blood pressure levels and the body fat percentage are measured while the mobile phone 168 is in an unfolded (open) state. The present embodiment also has the basic system structure for measuring the blood pressure and the body fat percentage and the structure of the sensing units 118 and 128 is the same as described above. Thus their description is hereby omitted.

FIG. 10 shows the back side of the mobile phone 168 according to the present embodiment, while in the open state. The first sensing unit 118 is disposed in a position in which the index finger of the left hand will be naturally placed when a person holds the mobile phone with both hands (when the mobile phone 168 is held facing the front face), and the second sensing unit 128 is disposed in a position in which the index finger of the right hand will be naturally placed when a person holds the mobile phone with both hands.

Thus, when measuring blood pressure and body fat percentage, the index finger of the left hand contacts the first sensing unit 118, and the index finger of the right hand contacts the second sensing unit 128 while the mobile phone 168 is open. Thus, the pulse wave signal of the left hand finger obtained by the first light receiving element 28B of the first sensing unit 118, and the pulse wave signal of the right hand finger obtained by the second light receiving element 38B of the second sensing unit 128 are transmitted to the blood pressure level measuring unit 78, wherein the blood pressure level is measured. Also, the body's impedance value measured between the first impedance sensing unit 48 of the first sensing unit 118 and the second impedance sensing unit 58 of the second sensing unit 128, and data such as the subject's weight, height, age and sex that was input from the numerical input unit 98, are transmitted to the body fat percentage measuring unit 68, wherein the body fat percentage is calculated by the body fat percentage measuring unit 68. The blood pressure and body fat percentage that are determined in this way are displayed on the display screen 88 of the mobile phone. The display screen 88 may be the display panel on the front side of the mobile phone, or it may be the display panel 818 on the back side (what is known as a sub-screen).

### Eighth Embodiment

The present embodiment is an embodiment in which the portable-type health management device is incorporated as a single piece in a non-flip-type mobile phone (what is known as a "straight-type mobile phone"). In the present embodiment as well, the basic system structure for measuring the blood pressure and the body fat percentage, and the structure of the sensing units 118 and 128 is the same as described above, and thus their description is hereby omitted.

FIG. 11 shows a front side of a mobile phone 268 according to the present embodiment. The first sensing unit 118 is disposed in a position in which the thumb of the left hand can be naturally placed when a person . holds the mobile phone with both hands. More specifically, the first sensing unit 118 is disposed in a position in the vicinity of the left side edge of the central portion in the longitudinal direction (the vertical direction of FIG. 11) or in a position in the vicinity of the left side edge of the upper end portion in the longitudinal direction of the mobile phone 268.

On the other hand, the second sensing unit 128 is disposed in a position in which the thumb of the right hand can be naturally placed when a person holds the mobile phone with both hands. More specifically, the second sensing unit 128 is disposed in a position in the vicinity of the right side edge of the central portion in the longitudinal direction (the vertical direction of FIG. 11), in a position in the vicinity of the right side edge of the upper end portion in the longitudinal direction, or in a position at the center, in the horizontal direction, at the top end portion in the longitudinal direction of the mobile phone 268.

Thus, the thumb of the left hand contacts the first sensing unit 118 and the thumb of the right hand contacts the second sensing unit 128 when measuring blood pressure and body fat percentage. Thus, the blood pressure level and the body fat percentage are measured, and the result is displayed on the display screen 88 of the mobile phone 268 in a similar manner to the embodiments described above.

It should be noted that in the case of the present embodiment, the locations in which the first sensing unit 118 and the second sensing unit 128 are disposed are not limited to a single location each, but may be disposed in multiple locations (the locations described above).

### Ninth Embodiment

The present embodiment is a modified embodiment of the case in which the portable-type health management device is incorporated as a single piece in the non-flip-type mobile phone 268 (what is known as a "straight-type mobile phone"). The basic system structure for measuring the blood pressure and the body fat percentage, and the structure of the sensing units 118 and 128 of the present embodiment is the same as described above, and thus their description is hereby omitted.

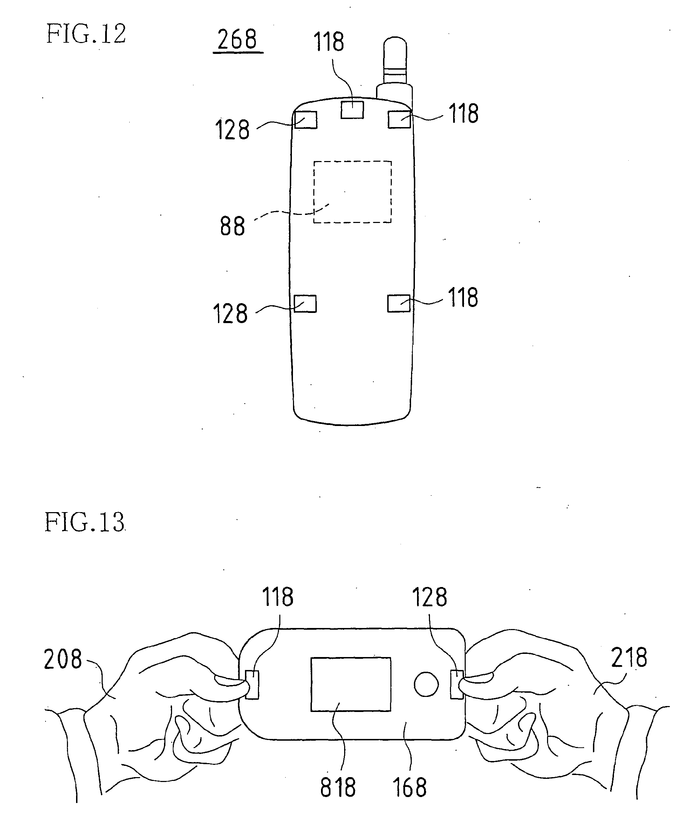

FIG. 12 shows the back side of the mobile phone 268 according to the present embodiment. The first sensing unit 118 is disposed in a position in which the index finger of the left hand can be naturally placed when a person holds the mobile phone with both hands. More specifically, the first sensing unit 118 is disposed on the back surface in a position in the vicinity of the right side edge of the central portion in the longitudinal direction (the vertical direction of FIG. 12), in a position in the vicinity of the right side edge of the upper end portion in the longitudinal direction, or in a position at the center, in the horizontal direction, of the top end portion in the longitudinal direction.

On the other hand, the second sensing unit 128 is disposed in a position in which the index finger of the right hand can be naturally placed when a person holds the mobile phone with both hands. More specifically, the second sensing unit 128 is disposed on the back surface in a position in the vicinity of the left side edge of the central portion in the longitudinal direction (the vertical direction of FIG. 12), or in a position in the vicinity of the left side edge of the upper end portion in the longitudinal direction.

Thus, the index finger of the left hand contacts the first sensing unit 118 and the index finger of the right hand contacts the second sensing unit 128 when measuring blood pressure and body fat percentage. Thus, the blood pressure level and the body fat percentage are measured and the result is displayed on the display screen 88 of the mobile phone 268, in a similar manner to the embodiments described above.

It should be noted that in the case of the present embodiment as well, the locations in which the first sensing unit 118 and the second sensing unit 128 are disposed are also not limited to a single location each, but may be disposed in multiple locations (the locations described above).

### Tenth Embodiment

The present embodiment is one in which an IrDA light emitting element, which is an infrared communication element mounted in a mobile phone, is used as the light emitting element of a sensing unit. That is, functions for both communicating and for pulse wave detection are included in the IrDA light emitting element. In this case, the wavelength of the light that is emitted by the light emitting element, and the wavelength of light to which the light receiving element is sensitive is 940 nm, and the IrDA light emitting element has an emitted light wavelength and a received light wavelength that are capable of measuring a pulse wave. Moreover, the IrDA light emitting element is formed as a single piece with the impedance sensing unit, and this may be taken as the sensing unit.

It should be noted that in a mobile phone in which the IrDA light emitting element is only provided in a single location, the IrDA device may be utilized as the light emitting element of either one of the pulse wave sensing units, and for a mobile phone in which an IrDA light emitting element is provided in two locations, the elements of the IrDA device may be used as the light emitting elements 28A and 38A of both pulse wave sensing units 28 and 38.

FIG. 13 shows a measuring operation using a flip-type mobile phone 168 containing an IrDA light emitting element, the mobile phone provided with a first sensing unit 118 in which the IrDA light emitting element is formed as a single piece with the impedance sensing unit.

As shown in FIG. 13, the sensing units 118 and 128 are disposed on both ends respectively, in the longitudinal direction of the mobile phone 168 while in the closed state. When measuring the blood pressure and body fat percentage, the thumb of the left hand 208 touches the first sensing unit 118, and the thumb of the right hand 218 touches the second sensing unit 128 while the mobile phone is in the closed state. Thus the blood pressure level and the body fat percentage are measured in a similar manner to the case of the embodiments described above. The blood pressure level and the body fat percentage that are determined in this way are displayed on the display screen 818 of the mobile phone (displayed on the sub-screen in the case of the flip-type mobile phone 168).

### Eleventh Embodiment

The present embodiment relates to a case in which the portable-type health management device, provided with a sensing unit in which an IrDA light emitting element is utilized as the light emitting element of the pulse wave sensing unit, and in which the pulse wave sensing unit and the impedance sensing unit are formed as a single piece, is incorporated as a single piece with the mobile phone 168, wherein the portable-type health management device is configured so as to measure blood pressure level and body fat percentage while the mobile phone 168 is in the open state.

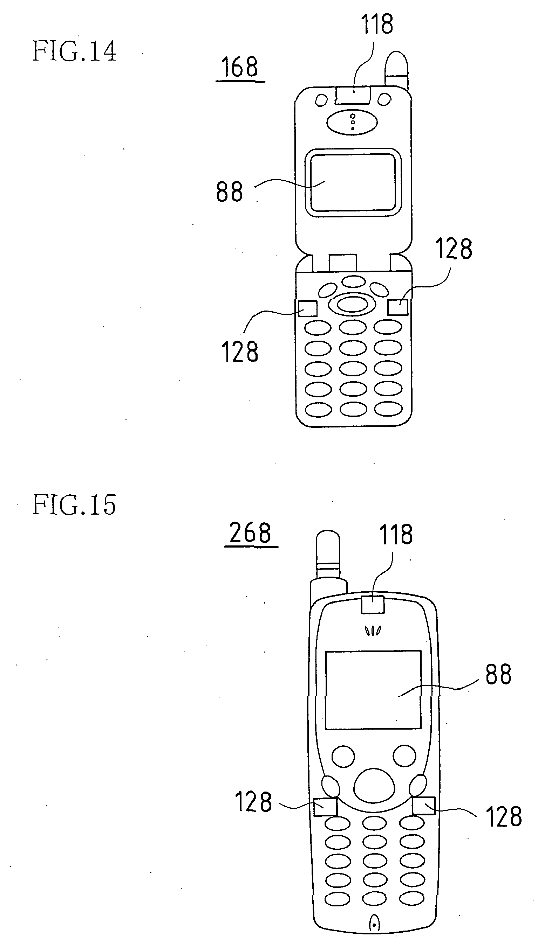

FIG. 14 shows the mobile phone 168 according to the present embodiment in the open state. The first sensing unit 118, in which the IRDA light emitting element and the impedance unit are formed as a single piece, is disposed in a position in which the thumb or the index finger of the left or the right hand is naturally placed when the mobile phone is held by a person, and the second sensing units 128 are disposed in positions in which the thumb or the index finger of the remaining hand is naturally placed when the mobile phone is held by a person (either one of the two location shown in FIG. 14).

Thus, when measuring the blood pressure and body fat percentage, the right or the left hand touches the first sensing unit 118, while the mobile phone 168 is in the open state, and the other hand touches the second sensing unit 128. Thus, the blood pressure level and the body fat percentage are measured in a similar manner as the embodiment described above, and the results are displayed on the display screen 88 of the mobile phone. It should be noted that the second sensing unit 128 may be provided at both of the two locations shown in FIG. 14.

### Twelfth Embodiment

The present embodiment relates to a case in which the portable-type health management device, provided with a sensing unit in which an IrDA light emitting element is utilized as the light emitting element of the pulse wave sensing unit, and in which the pulse wave sensing unit and the impedance sensing unit are formed as a single piece, is incorporated as a single piece with the mobile phone 268 (what is known as a "straight type mobile phone").

FIG. 15 shows the mobile phone 268 according to the present embodiment. The first sensing unit 118 in which the IRDA light emitting element and the impedance sensing unit are formed as a single piece is disposed in a position in which the thumb or the index finger of the left or the right hand is naturally placed when the mobile phone is held by a person, and the second sensing units 128 are disposed in positions in which the thumb or the index finger of the remaining hand is naturally placed when the mobile phone is held by a person (either one of the two location shown in FIG. 15).

Thus, when measuring the blood pressure and body fat percentage, the right or the left hand touches the first sensing unit 118, and the other hand touches the second sensing unit 128. Thus, the blood pressure level and the body fat percentage are measured in a similar manner to the embodiments described above, and the results are displayed on the display screen 88 of the mobile phone. It should be noted that in the present embodiment, the second sensing unit 128 may also be provided at both of the two locations shown in FIG. 15.

### Thirteenth Embodiment

The present embodiment relates to a case in which, in a mobile phone 168 provided with a fingerprint sensor and an IrDA light emitting element, the portable-type health management device provided with a sensing unit in which an IrDA light emitting element is utilized as the light emitting element of the pulse wave sensing unit, and in which the pulse wave sensing unit and the impedance sensing unit are formed as a single piece, is incorporated as a single piece in the flip-type mobile phone 168, and the blood pressure level and body-fat percentage are measured while the mobile phone 168 is in the unfolded (open) state.

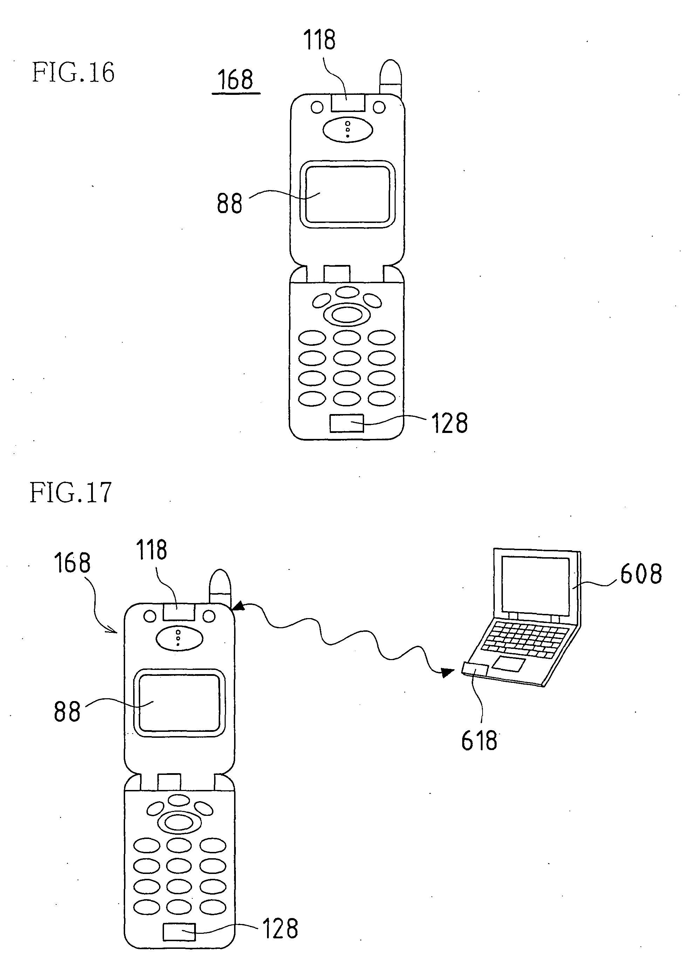

FIG. 16 shows the mobile phone 168 according to the present invention in the unfolded state, wherein the first sensing unit 118 in which the IRDA light emitting element and the impedance unit are formed as a single piece, is disposed in a position in which the thumb or the index finger of the left or the right hand is naturally placed when the mobile phone is held by a person, and the second sensing unit 128, which serves as the electrodes of the fingerprint sensor and the electrodes of the impedance sensing unit, is disposed in a position in which the thumb or the index finger of the remaining hand is naturally placed when the mobile phone is held by a person.

Thus, when measuring the blood pressure and the body fat percentage, the left hand or the right hand contacts the first sensing unit 118 while the mobile phone 168 is in the open state, and the other hand touches the second sensing unit 128. Thus, in a similar manner to the above-noted embodiments, the blood pressure level and the body fat percentage is measured, and the result is displayed on the display screen 88 of the mobile phone.

### Fourteenth Embodiment

The fourteenth embodiment will be described next. As shown in FIG. 17, the present embodiment is one that uses a mobile phone 168 that is capable of infrared communications and that contains a built-in first sensing unit 118 in which the IrDA light emitting element is utilized as the light emitting element of the pulse wave sensing unit, and which is formed as a single piece with the impedance unit, and a second sensing unit 128. The portable-type health management device is configured to perform infrared communication between the mobile phone 168 and an external device (personal computer) 608 that is provided with an in-built IrDA device 618 and that is capable of infrared communication in which the IrDA device is utilized, to send the blood pressure levels and the body fat percentage measured with the mobile phone 168 to the external device 608 for performing daily health management, and to send information necessary for measuring the blood pressure level and the body fat percentage, such as height, weight, age, sex and reference blood pressure from the external device 608 to the mobile phone 168.

### Fifteenth Embodiment

For a mobile phone 168 provided with an IrDA light emitting element, the present embodiment relates to a case in which a portable-type health management device provided with a sensing unit in which the IrDA light emitting element is utilized as the light emitting element of the pulse wave sensing unit, and in which the pulse wave sensing unit is formed in a single piece with the impedance sensing unit, is incorporated as a single piece into the flip-type mobile phone 168, wherein the blood pressure level, body-fat percentage and blood sugar level are measured while the mobile phone 168 is in the unfolded (open) state.

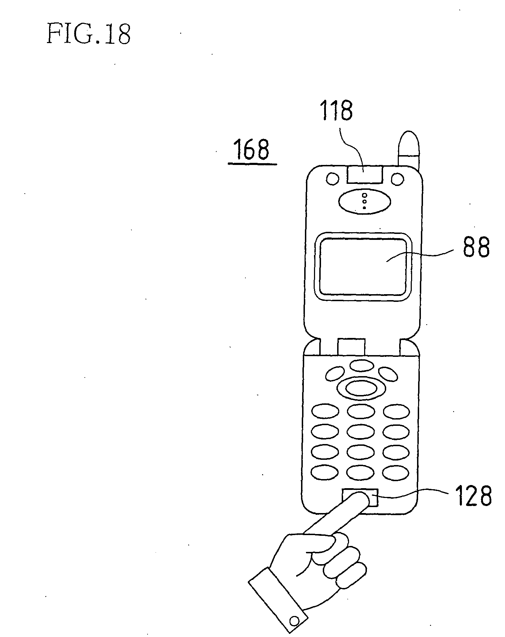

FIG. 18 shows the mobile phone 168 according to the present embodiment in the unfolded state, wherein the first sensing unit 118, made by forming the IRDA light emitting element and the impedance unit in a single piece, is disposed in a position in which the thumb or the index finger of the left or the right hand is naturally placed when the mobile phone is held by a person, and the second sensing unit 128, which serves both as a blood sugar level sensor and as the electrodes of the impedance sensing unit, is disposed in a position in which the thumb or the index finger of the remaining hand is naturally placed when the mobile phone is held by a person.

Thus, when measuring the blood pressure, the body fat percentage and the blood sugar level, the left hand or the right hand contacts the first sensing unit 118 while the mobile phone 168 is in the open state, and the other hand touches the second sensing unit 128. Thus, the blood pressure level and the body fat percentage are measured in a similar manner to the above-noted embodiments. Additionally, the blood sugar level is also measured, and the results are displayed on the display screen 88 of the mobile phone. Moreover, the measured blood pressure, body fat percentage and blood sugar level are sent to the external device 608 for performing daily health management by infrared communication using the IrDA light emitting element, and information necessary for measuring the blood pressure level, body fat percentage and blood sugar level, such as height, weight, age, sex and reference blood pressure can also be sent from the external device 608 to the mobile phone 168.

It should be noted that the blood sugar level sensor of the present embodiment may be a well-known chemical type blood sugar level sensor, or an optical type blood sugar level sensor.

Furthermore, in the present embodiment, it is also possible to configure the portable-type health management device so as to transmit the medical information, such as the measured blood sugar level, blood pressure and body fat percentage, to a medical institution or the like using a communicating function such as the internet built into the mobile phone 168, to have a doctor make a diagnosis, and then send the result of the diagnosis back to the mobile phone 168 using the communicating function such as the internet. Moreover, it is also possible to make an appointment to visit the medical institution.

In a mobile phone that has an inbuilt function to execute application software, it is also possible to execute an application based on the medical information, such as the measured blood sugar level, blood pressure and pulse rate. For example, it is possible to alter the progress of a game in accordance with changes in blood pressure or pulse rate, or to assess compatibility with the other party when using a video phone based on changes in blood pressure and pulse rate. It is also possible to execute applications, such as those for diagnosing the degree of stress, based on blood pressure and pulse rate. These are not limited to the mobile phone 168 according to the present invention, and can also be applied to any of the mobile phones according to the above-noted embodiments.

### Sixteenth Embodiment

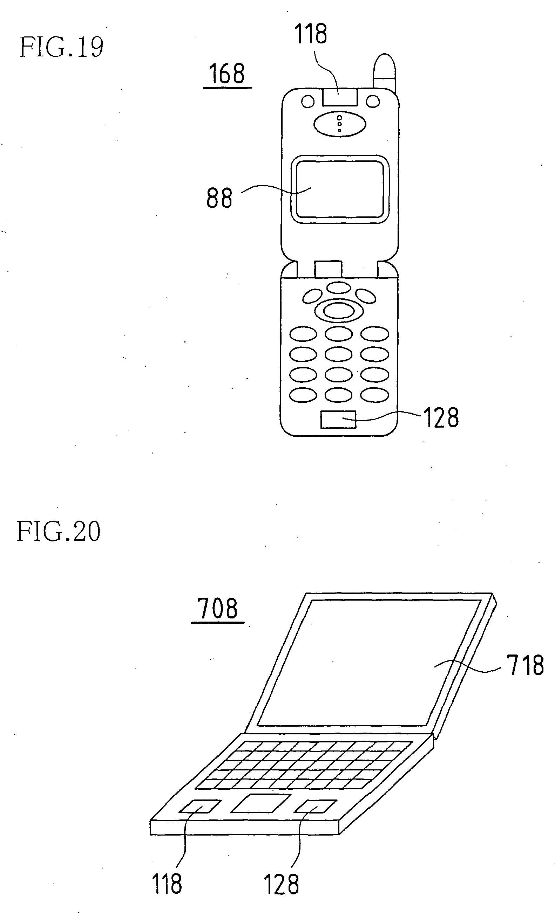

The sixteenth embodiment will be described next. The present embodiment is one in which a mobile phone 168 that contains an inbuilt alarm function (FIG. 19) incorporates the portable-type health management device according to the present invention, wherein the time for measuring the medical information is set with the alarm function. Thus the medical information can be measured every day at a specified time without being forgotten.

### Seventeenth Embodiment

The seventeenth embodiment will be described next. The present embodiment is one in which the portable-type health management device is incorporated as a single piece with a notebook-type personal computer, which is provided as a mobile information terminal device.

In a personal computer 708 as shown in FIG. 20, the first sensing unit 118 is disposed in a position to which the left hand will naturally come when a person types at a keyboard, and the second sensing unit 128 is disposed in a position to which the right hand will naturally come when a person types at the keyboard.

Thus, when measuring blood pressure and body fat percentage the index finger, the thumb, or the palm, for example, of the left hand touches the sensing unit 118, and the index finger, the thumb or the palm, for example of the right hand touches the second sensing unit 128. Thus, the blood pressure level and the body fat percentage are measured in the same manner as in the cases of the embodiments noted above, and those results are displayed on a liquid screen panel 718 of the personal computer 708.

### Eighteenth Embodiment

The eighteenth embodiment of the present invention will be described next. The present embodiment is one in which the portable-type health management device is incorporated as a single piece with a mouse that is connected to a personal computer, which is provided as a mobile information terminal device.

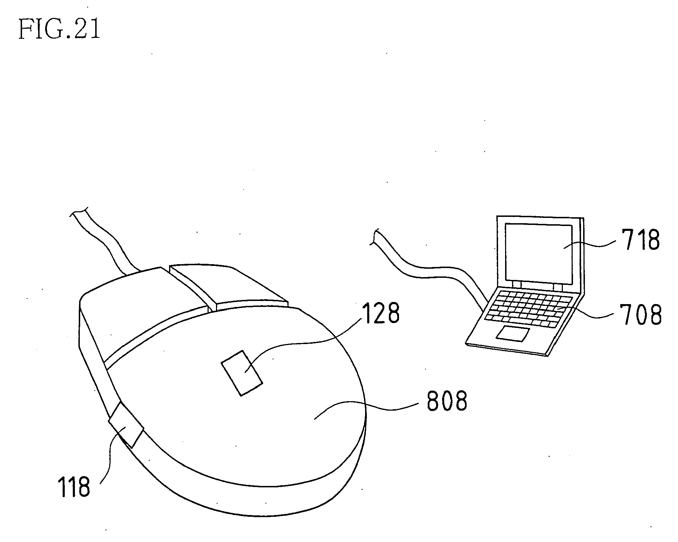

As shown in FIG. 21, the second sensing unit 128 is disposed in the vicinity of an upper portion of a mouse 808, and the first sensing unit is disposed on a side face of the mouse 808.

Thus, when measuring blood pressure and body fat percentage, the palm, the thumb or the index finger, for example, of the right hand contacts the second sensing unit 128, and the thumb or the index finger, for example, of the left hand contacts the first sensing unit 118. Thus, the blood pressure level and the body fat percentage are measured in the same way as the cases of the above-noted embodiments and the result is displayed on the liquid crystal display panel 728 of the personal computer 708.

A case in which a steering wheel, shift lever or seat, for example, which are provided as internal fixtures of a vehicle, is utilized to constitute a medical information detection apparatus (referred to below as a vehicle-mounted health management device) as an embodiment of the medical information detection apparatus of the present invention will be described next.
Further embodiments of the present invention are described below with reference to the drawings.

### Basic System Structure of the Vehicle-Mounted Health Management Device

With the specific configuration of the vehicle-mounted health management device (the configuration in which the vehicle-mounted health management device is incorporated as a single piece in the steering wheel or the like), the basic system for measuring blood pressure and body fat percentage is the same as the configuration of FIG. 6 described previously, apart from the fact that the numerical input unit 98 is not only capable of directly inputting data such as the subject's weight, height, age and sex, in the configuration shown in FIG. 6, but that in the present embodiment, this data can be received (received from an external device 30, which will be described later).

With this configuration, the impedance value of the human body measured by the impedance sensing unit 48 and 58 and the data, such as the subject's weight, height, age and sex that is input from the numerical input unit 98 (or that the numerical input unit 98 receives) is transmitted to the body fat percentage calculating unit 68, and the body fat percentage is calculated in the body fat percentage calculating unit 68.

The information of the blood pressure levels and the body fat percentage determined in this way is transmitted to the display unit 88 and is displayed in the display unit 88 so that the subject can visually confirm it, or is displayed on a display screen of an external device 308 by information transfer from the display screen 88 to the external device 308.

The configuration of the sensing unit is also the same as in FIG. 7A and FIG. 7B, and thus the description thereof is hereby omitted.

### How the Vehicle-Mounted Health Management Device is Incorporated into the Steering Wheel

The configuration and measurement operation of a case in which the vehicle-mounted health management device, provided with the above noted basic system configuration, is incorporated into a steering wheel is described next.

### Nineteenth Embodiment

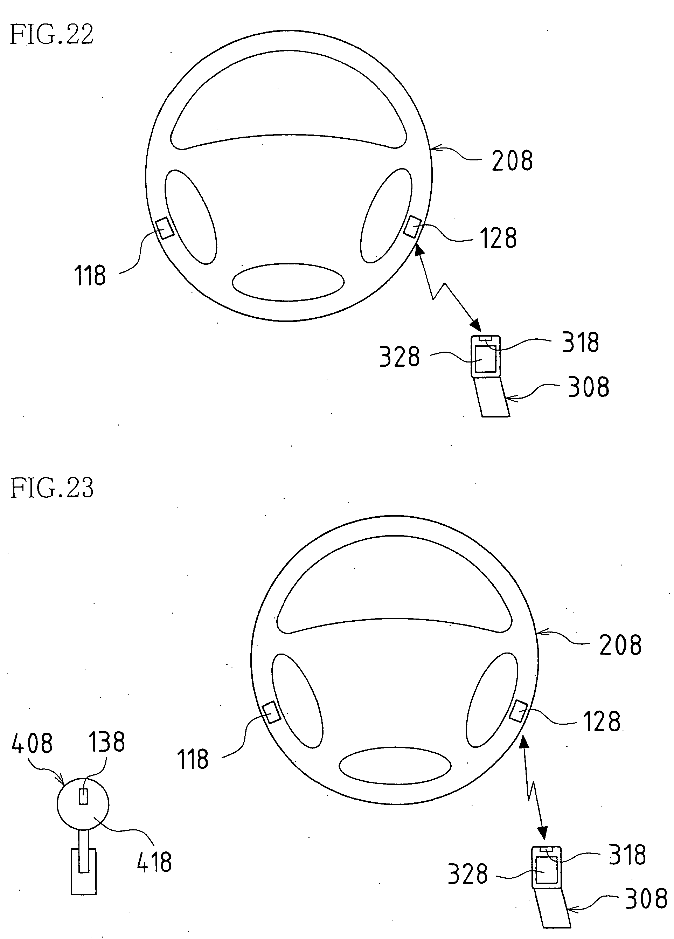

FIG. 22 shows a steering wheel 208, and an external device 308 (such as a mobile phone) that is capable of communicating with the vehicle-mounted health management device incorporated in the steering wheel 208, according to the nineteenth embodiment.

As shown in FIG. 22, the sensing units 118 and 128 are each provided on parts of the steering wheel 208 that are gripped by the driver. More specifically, the first sensing unit 118 is embedded in a lower left portion when the steering wheel 208 is viewed from the front (what is known as the 8 o'clock position), and the second sensing unit 128 is embedded in a lower right portion when the steering wheel is viewed from the front (what is known as the 4 o'clock position).

The second light emitting element 38A of one of those sensing units 118 and 128 (for example, the second sensing unit 128) is constituted by an IrDA device, serving as an infrared communication element, wherein the vehicle-mounted health management device is configured such that information of the measured blood pressure level and body fat percentage is transmitted to the external device 308 by infrared communication with the IrDA device. Thus, an IrDA device 31 for infrared communication is also provided on the external device 308. Moreover, the external device 308 stores the information necessary for measuring blood pressure and body fat percentage, and is also configured so as to transmit this information to the vehicle-mounted health management device. This transmitted information may be taken to be information such as the subject's weight, height, age, sex and reference blood pressure.

While driving the vehicle, the driver's left hand contacts the first sensing unit 118 and the driver's right hand contacts the second sensing unit 128. Thus, the pulse wave signal of the left hand that is obtained by the first light receiving element 28B of the first sensing unit 118, and the pulse wave signal of the right hand that is obtained by the second light receiving element 38B of the second sensing unit 128 are transmitted to the blood pressure level calculating unit 78 where the blood pressure level is calculated. The body's impedance value that is measured between the first impedance sensing unit 48 of the first sensing unit 118 and the second impedance sensing unit 58 of the second sensing unit 128, and the data, such as the subject's weight, height, age and sex that is input from the numerical input unit 98 (or that is received from the external device 308) is transmitted to the body fat percentage measuring unit 68, and the body fat percentage is calculated in the body fat percentage measuring unit 68. Information of the blood pressure levels and body fat percentage information determined in this way is transmitted to the external device 308, and the information is displayed on a display panel 328 provided on the external device 308 and is stored. Furthermore, the blood pressure level, and value of the body fat percentage may also be displayed on an instrument panel in the vehicle.

### Twentieth Embodiment

In addition to the configuration of the above-noted nineteenth embodiment, the present embodiment is one in which a sensing unit is provided on the shift lever. That is to say, in addition to the first and second sensing units 118 and 128 provided on the steering wheel 208, the present embodiment has a third sensing unit 138 embedded in a grip portion 418 of a shift lever 408 as shown in FIG. 23. The configuration of the third sensing unit 138 is substantially the same as the first sensing unit 118 of the first embodiment described above, and so the description thereof is hereby omitted.

In the configuration of the present embodiment, the driver has a driving posture in which, when driving the vehicle, the left hand contacts the first sensing unit 118 and the right hand contacts the second sensing unit 128, or a posture in which the left hand contacts the shift lever 408 and the right hand contacts the second sensing unit 128.

In the case of the first driving posture, the blood pressure and body fat percentage can be measured in the same way as in the case of the above-described nineteenth embodiment.

On the other hand, in the case of the second driving posture, the pulse wave signal of the left hand that is obtained by the light receiving element of the third sensing unit 138 and the pulse wave signal of the right hand that is obtained by the second light receiving element 38B of the second sensing unit 128 are transmitted to the blood pressure measuring unit 78, wherein the blood pressure level is measured. Furthermore, the body's impedance value that is measured between an impedance sensing unit of the third sensing unit 138 and the second impedance sensing unit 58 of the second sensing unit 128, and the data, such as the subject's weight, height, age and sex that is input from the numerical input unit 98 (or that is received from the external device 308) is transmitted to the body fat percentage measuring unit 68, wherein the body fat percentage is calculated in the body fat percentage measuring unit 68. The blood pressure levels and body fat percentage information determined in this way are transmitted to the external device 308, and the information is displayed on a display panel 328 provided on the external device 308 and is stored. Furthermore, the blood pressure level, and value of the body fat percentage may also be displayed on an instrument panel in the vehicle.

### Twenty-first Embodiment

The present embodiment is one in which, in addition to the configuration of the twentieth embodiment described above, a blood sugar level sensor is provided in each of the sensing units 118, 128 and 138, whereby blood pressure, body fat percentage and blood sugar level can be measured.

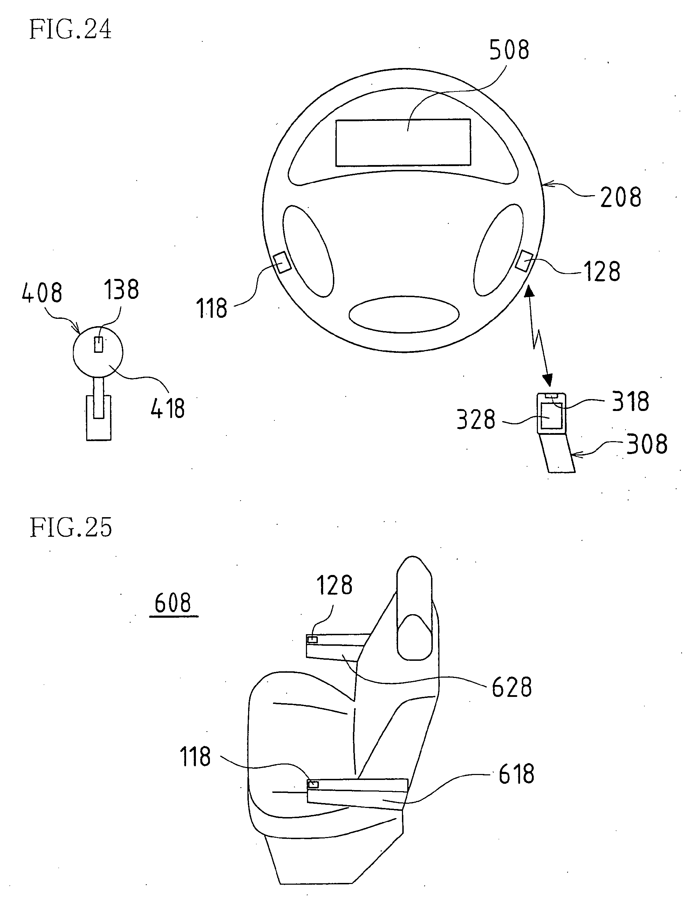

As shown in FIG. 24, in the configuration of the present embodiment, the information of the measured blood pressure level, body fat percentage and blood sugar level is transmitted to the external device 308, and the information is displayed on the display panel 38 provided on the external device 308, and is stored. Furthermore, the blood pressure level, body fat percentage and blood sugar level may also be displayed on an instrument panel 508 in the vehicle.

It should be noted that the blood sugar level sensor of the present embodiment may be a well-known chemical blood sugar level sensor, or optical blood sugar level sensor.

### Twenty-second Embodiment

The configuration and measurement operation of a case in which the vehicle-mounted health management device provided with the above-noted basic system configuration is incorporated into a seat in the vehicle is described next.

FIG. 25 shows a seat 608 according to the present twenty-second embodiment. As shown in FIG. 25, the first sensing unit 118 is embedded in an armrest 618 for the left hand, provided on the seat 608, and the second sensing unit 128 is embedded in an armrest 628 for the right hand, provided on the seat 608. The positions in which the sensing units 118 and 128 are embedded are set as positions in which the subject's palms or finger tips make contact when the subject's arms are placed on the armrests 618 and 628, when sitting in the seat 608.

In the case of the present embodiment too, the vehicle-mounted health management system is configured such that the second light emitting element 38A of one of the sensing units 118 and 128 (for example, the second sensing unit 128) is constituted by an IrDA device serving as an infrared communication element, wherein the information of the measured blood pressure level and body fat percentage is transmitted to an unshown external device by infrared communication with the IrDA device. The external device is the same as the external device in the embodiments described above, and so the description thereof is hereby omitted.

When a passenger in the vehicle sits in the seat 608 and places his arms on the armrests 618 and 628, the left hand contacts the first sensing unit 118, and the right hand contacts the second sensing unit 128. Thus, the pulse wave signal of the left hand that is obtained by the first light receiving element 28B of the first sensing unit 118, and the pulse wave signal of the right hand that is obtained by the second light receiving element 38B of the second sensing unit 128 are transmitted to the blood pressure level measuring unit 78 wherein the blood pressure level is measured. The body's impedance value that is measured between the first impedance sensing unit 48 of the first sensing unit 118 and the second impedance sensing unit 58 of the second sensing unit 128, and the data, such as the subject's weight, height, age and sex that is input from the numerical input unit 98 (or that is received from the external device 308) is transmitted to the body fat percentage measuring unit 68, and the body fat percentage is calculated in the body fat percentage measuring unit 68. The information of the blood pressure level and body fat percentage determined in this way is transmitted to the external device 308, and the information is displayed on a display panel 328 provided on the external device 308 and is stored. Furthermore, the blood pressure level, and value of the body fat percentage may also be displayed on an instrument panel in the vehicle.

It should be noted that the seat according to the present embodiment may be applied to any one of the driver's seat, the passenger's seat, or the back seats.

The vehicle-mounted health management device according to the embodiments described above has been provided so as to only display the measurement results, however for the vehicle mounted health management device according to the embodiments below, the vehicle responds in accordance with the measured values.

### Twenty-third Embodiment

The present embodiment is one in which the vehicle-mounted health management device judges whether or not the subject is in a physical condition to drive the vehicle, based on the measured values, and is configured so as to notify the result of the judgment.

More specifically, based on the measured values, the vehicle-mounted health management device judges whether or not the subject is sleepy, whether or not the pulse rate is much faster than normal, whether or not blood pressure is much higher than normal and whether or not the subject's consciousness is muddled or is absent due to diabetes, wherein warnings such as "danger" may be displayed on the instrument panel in response. The subject may also be warned by an alarm sound. These control operations may be executed by unshown notifying means provided in the vehicle-mounted health management device. Furthermore, in a situation in which the subject drives the vehicle despite not being in a condition to do so, the present embodiment may be set so as to automatically stop the vehicle.

### Twenty-fourth Embodiment

In the present embodiment, the vehicle mounted health management device is configured so as to adjust the set strength and set temperature of an air-conditioning apparatus based on the measured values.

More specifically, the vehicle mounted health management device adjusts the temperature, strength, direction and humidity of the air in the air-conditioning apparatus, for example, wherein when the blood pressure of the subject is high, the air-conditioning apparatus is adjusted to increase the wind strength and decrease the set temperature of the air blown onto the subject. For example, if the seat 608 according to the twenty-second embodiment described above is used as the driving seat, the passenger seat, and the back seats respectively, then it is possible to set the state of the air-conditioning individually in accordance with the condition of each passenger. These control operations may be executed by unshown air-conditioning controlling means provided in the vehicle mounted health management device.

### Twenty-fifth Embodiment

In the present embodiment, the vehicle mounted health management device is configured so as to adjust the volume of an acoustic device based on the measured values. More specifically, if the subject's blood pressure is measured, then the vehicle mounted health management device can be set to increase the volume of the acoustic device if it is judged that the subject is getting sleepy.

Furthermore, in a case in which the seat 608 according to the twenty-second embodiment described above is used for the driving seat, the passenger seat, and the back seats respectively, if a passenger other than the driver is judged to be getting sleepy, then it is possible to reduce the volume of the sound coming from the speaker that is closest to the seating position of that passenger. These control operations may be executed by unshown acoustic controlling means provided in the vehicle mounted health management device.

It should be noted that, in the embodiments described above, one each of the first and second sensing units 118 and 128 have been disposed on the steering wheel 208, however it is also possible to dispose a plurality of each of the sensing units.

### Twenty-Sixth Embodiment

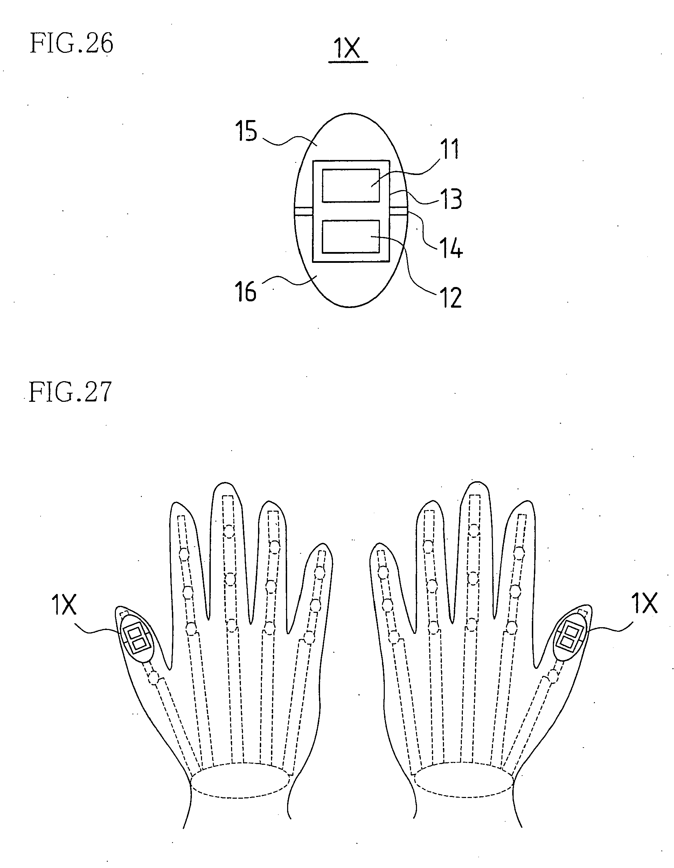

The twenty-sixth embodiment is described next with reference to the drawings. FIG. 26 shows a structural example of a sensor unit 1X for sensing medical information.

The sensor unit 1X of the present embodiment is capable of sensing medical information by attachment to the finger tips of a hand portion of a robot arm, as shown in FIG. 27, for example. In this example, the sensor units 1X are attached on the palm side of the thumbs of the left and right hands, however there is no limitation to attaching to sensor units 1X to just the thumbs, and they may be attached to the index fingers, middle, ring or little fingers, or the like. There is also no limitation to attach the sensor unit 1X to the finger tips, and so they may, in some cases, be attached to the back of the hand or the palm. In any case, the sensor units 1X should be attached in positions that are easiest for sensing a person's medical information.

The sensor unit 1X shown in FIG. 26 is provided with a light emitting device 11 and a light receiving device 12, serving as pulse wave sensors for measuring information such as a person's pulse rate and blood pressure, mounted on a flexible substrate 13 and arranged in the center, and electrodes 15 and 16, divided into two by an insulating portion 14, provided so as to surround the light emitting device 11 and the light receiving device 12. The electrodes 15 and 16 are the current application electrode and the voltage detection electrode for measuring a person's impedance, and they can also serve as electrodes for measuring electrocardiac signals.

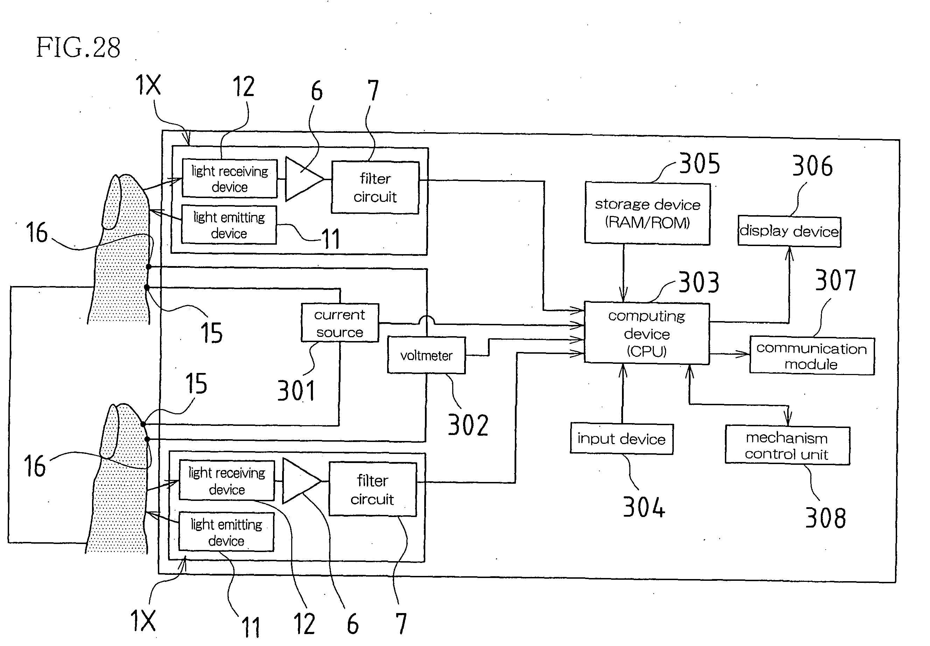

FIG. 28 is a block diagram showing a system configuration of a robot-type medical information detection apparatus that contains a sensor unit 1X of the configuration described above.

First, since it is necessary to sense the pulse waves at a plurality of locations in order to measure blood pressure, in this example, two of the sensor units 1X shown in FIG. 26 are used, and the sensor units 1X are disposed on the finger tips (in this example, the thumbs) of the hands of the left and right arms. The outputs of the light receiving devices 12 arranged in this way pass through amps 6 and filter circuits 7, after which they are input to a computing unit 303, which will be described later.

In this embodiment, a current source 301 for applying an electric current to the current application electrodes 15, a voltmeter 302 for measuring the voltage between the two voltage detecting electrodes 16, the computing unit (CPU) 303, an input device 304, a storage device (RAM/ROM) 305, a display device 306 and a communication module 307, and the like are provided as a measurement system. A mechanism control unit 308 for controlling various mechanisms such as a robot arm, a hand or fingers is connected in both directions to the computing unit 303.

### Twenty-seventh Embodiment

The embodiment of a case in which the robot-type medical information detection apparatus having the above described configuration is used as a medical / care robot is described next. It is believed that in the aging society to come, there will be developments in robots for medical treatment and senior care. Of the medical information about a person, information such as a person's pulse rate and blood pressure is particularly important.

The measurement process of the twenty-seventh embodiment is described next.

First, in the present embodiment, information such as the systolic pressure and diastolic pressure values, which have been measured by another sphygmomanometer, age, height, weight and sex are input by the input device 304 and are stored in the storage device 305. The sensor units 1X are then activated while the two sensor unit 1X are in contact with a person's left and right finger respectively, as shown in FIG. 28, and the measurement is executed by applying a current to the current application electrodes 15 from the current source 301. A pulse wave signal from the two light receiving devices 12, a voltage signal measured in the voltmeter 302, and an applied current value from the current source 301 are input into the computing unit 303 in accordance with the measurement.

In the computing unit 303, the pulse rate and the pulse-wave propagation time (the time difference due to the difference in pulse-wave propagation velocity) are determined from the two pulse wave signals that are received, and a person's impedance value is determined based on the signals from the voltmeter 302 and the current source 301. The blood pressure levels (systolic pressure and diastolic pressure) are then determined from the pulse-wave propagation time. Or the blood pressure levels (systolic pressure and diastolic pressure) are determined by the time delay between the electrocardiac signals and the pulse wave. Techniques for determining blood pressure levels from electrocardiac signals and a pulse wave are disclosed in Japanese Patent No. 3028601 as described above. Furthermore, an inductive method has conventionally been used as the method for measuring electrocardiac signals. As the induction method, there is both bipolar induction and unipolar induction, but unipolar induction is used in the present embodiment. That is to say, one of the sensor units 1X is placed near to the heart and the other sensor unit 1X is placed in a region that is a distance from the heart (such as on a hand or foot) to measure weak electric current within the body, and thus measure the electrocardiac signals (myocardial power).

The computing unit 303 determines the body fat percentage or bone density based on the impedance values and the previously entered information such as age, height, weight and sex.

It should be noted that by emitting light of a plurality of appropriate wavelengths from the two light emitting devices 11 (a plurality of wavelengths of near infrared light), it is possible to measure the concentration of oxygen in the blood or the blood sugar level at the same time as well, based on analysis of the signals from the light receiving devices 12, which are capable of receiving near infrared light.

Furthermore, although not shown, if the sensor unit 1X is a pyroelectric sensor, then it is also possible to measure body temperature by placing the robot's thumb beside the ear to measure inside the ear with the pyroelectric sensor.

The computing unit 303 displays these calculated results on the display device and stores them in the storage device 305. Furthermore, the robot-type medical information detection apparatus may also be configured such that the calculated results such as electrocardiac signal, blood pressure (systolic pressure and diastolic pressure), pulse rate, pulse wave, body temperature, body fate, bone density, blood oxygen concentration and blood sugar level are transmitted and received to and from another external device using a communication module 307.

As described above, it is possible for a robot that imitates a human hand on which sensor units 1X are attached in the vicinity of the tips of, for example, the thumbs or the index fingers, to sense medical information by holding hands with a person. By providing the robot-type medical information detection apparatus in such a form, there is an advantage in that it is possible for the robot-type medical information detection apparatus to sense the medical information even when assisting the patient to walk.

It should be noted that in the twenty-seventh embodiment, the pulse wave was sensed by an optical method that uses the light emitting device 11 and the light receiving device 12, however if only the pulse wave is to be sensed, then it is also possible to use a pressure-type method for sensing the pressure fluctuations within the blood vessels with a pressure sensitive element.

### Twenty-eighth Embodiment

The twenty-eighth embodiment is an embodiment of a case in which the robot-type medical information detection apparatus of the present invention is used as an industrial robot.

Robots installed on manufacturing floors where there are both people and robots present carry out work while monitoring people's movement with a multitude of different sensors. However, with conventional robots that can obtain medical information only by sight or by touch, there is basically no contact between the robots because they communicate between each other via communication functions, and the like. But, in the case of a person entering amongst the robots, if the person does not move, then there is a high possibility that the robots cannot distinguish between the person and objects with thermal sensing, due to the sensor malfunctioning or due to reasons relating to the ambient temperature. Accordingly, by setting up industrial robots to be robots that have a function capable of sensing medical information, as in the present invention, it is possible to judge whether an object is a living organism or not using a touch sensor and the pulse wave sensor, and thus it is possible to prevent contact accidents between robots and people.

### Twenty-ninth Embodiment

The twenty-ninth embodiment is an embodiment of a case in which the robot-type medical information detection apparatus of the present information is used as a pet-type robot or a recreational robot.

As pet-type robots are becoming more popular, robots that can respond better to a person's physical or mental condition are becoming highly functional. Thus, by attaching a sensor unit 1X according to the present invention, pet-type robots are capable of checking their master's physical condition when they are held by their master. A suitable program can then be selected based on this information, and the pet-type robot may act in a way that pleases its master. Furthermore, it is possible to configure the pet-type robot to alert its master if the sensed value is abnormal, and if there is no reaction from the master, then to notify someone of the fact that there is an emergency using a network function.

In a similar way, a recreational robot may be able to provide an optimum service by selecting a program by ascertaining a person's physical condition.

An embodiment of a health management system of the present invention is described next with reference to the drawings.

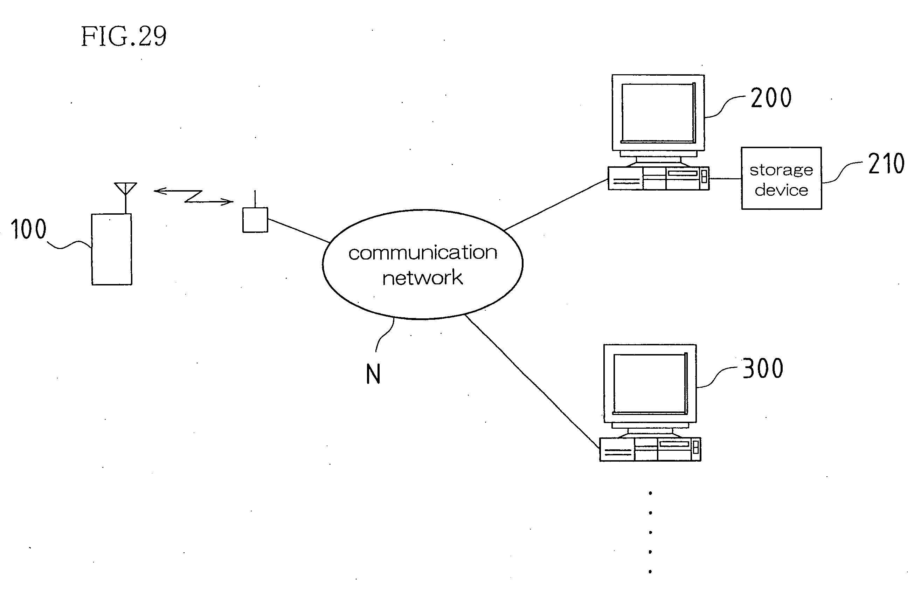

FIG. 29 is a block diagram showing a structural overview of one embodiment of the health management system of the present invention.

The health management system of the present embodiment is provided by a portable-type health management terminal 100 that is provided with a communication function, a management device 200 in a health management center, and a terminal 300 of facilities that can provide a necessary service to a service user who is carrying the health management terminal 100, wherein these can be connected via a communication network N (such as a mobile phone network, the internet or a dedicated phone line).

The health management terminal 100 is provided with a sensor unit 1X that can sense medical information.

Since the sensor unit 1X has the same configuration as the sensor unit 1X previously described and shown in FIG. 26, and the system structure of the medical information detection apparatus that has the sensor unit 1X is the same as the fourth embodiment (FIG. 5), the description of these is hereby omitted.

In the present embodiment, the medical information detection apparatus having the configuration described above is mounted on a mobile phone (health management terminal), which is an object that the service user carries every day. Consequently, in addition to a phone function, the communication module 37 is also provided with a mail function. However, alternatively, it is also possible that the object to be mounted may be a ring-type sensor that also serves as a watch or armband, or a necklace, for example.

Furthermore, with regards to measuring with the medical information detection apparatus, this is the same as with the fourth embodiment, but bone density may also be determined in the same way as body fat percentage. It should be noted that by emitting light of a plurality of appropriate wavelengths from the two light emitting devices 11 (a plurality of wavelengths of near infrared light), and measuring the degree of absorption of light by the oxy-hemoglobin in the blood, it is also possible to measure the blood oxygen concentration in the blood or the blood sugar level at the same time as well, based on analysis of the signals from the light receiving devices 12, capable of receiving near infrared light.

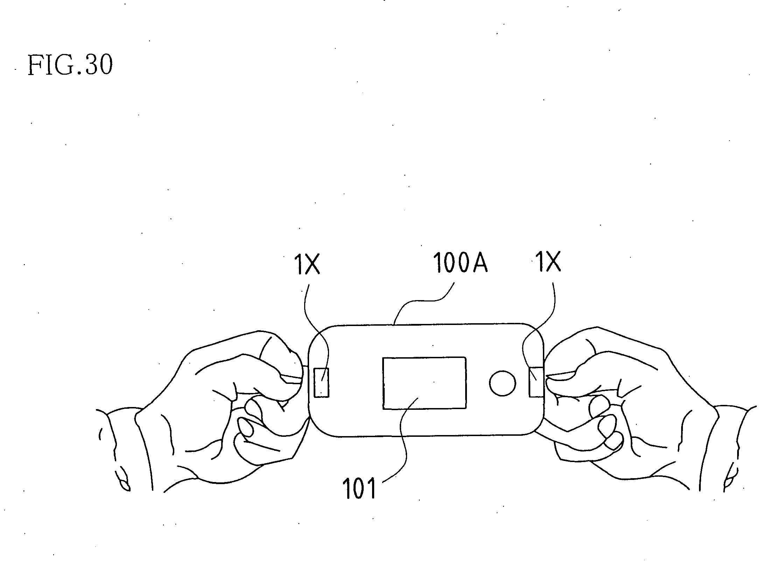

FIG. 30 shows an example of a layout configuration in a case in which two sensor unit 1X are disposed on a flip-type mobile phone 100A. In this example, the medical information detection apparatus is configured so as to sense the medical information while the mobile phone 100A is in a closed state. Thus, the two sensor units 1X are arranged on both sides, in the longitudinal direction, with a display unit 101 provided between them on the outer display face side of the main unit. Accordingly, the medical information detection apparatus is configured such that if the mobile phone 100A is gripped with both hands on the left and right side in the horizontal direction while in the closed state, then the tips of the thumbs of both hands will naturally contact each of the sensor units 1X.

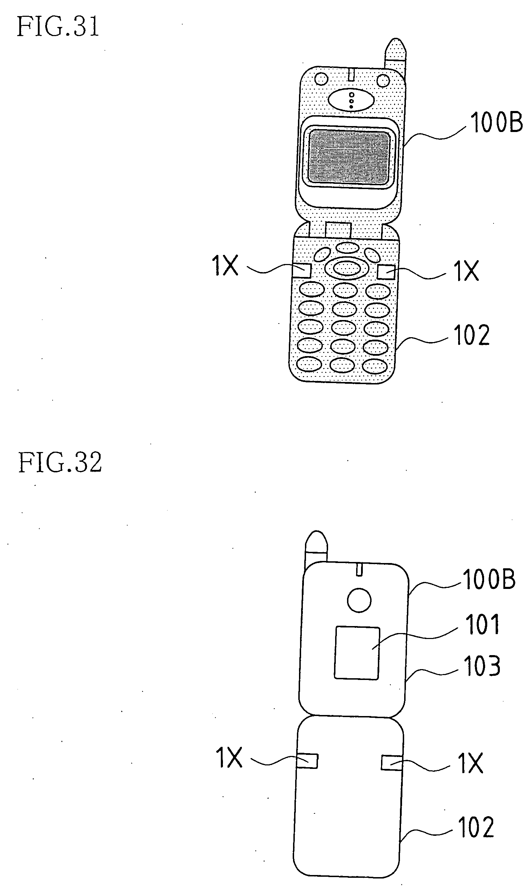

FIG. 31 and FIG. 32 show other layout configuration examples in a case in which the two sensor units 1X are disposed on a flip-type mobile phone 100B. In this example, the medical information is sensed while the mobile phone 100B is in the open state. Thus, the two sensor units 1X are disposed on the side 102 of the main unit on which the numeric keys are disposed. In FIG. 31, the sensor units 1X are arranged on an upper portion of the operating face side, on which the numeric keys on the main unit side 102 are disposed, and in FIG. 32, the sensor units 1X are arranged on an upper portion on both the left and right sides on a face that is the opposite side to the operating face side, on which the numeric keys of the main unit side 102 are disposed. Thus, in FIG. 31, the medical information detection apparatus is configured such that the service user's thumb and index finger naturally contact each of the sensor units 1X when the service user makes a call or answers the phone. Furthermore, in a similar manner to FIG. 30, in FIG. 32, the medical information detection apparatus is configured such that if the mobile phone 100B is gripped with both hands on the left and right side, with respect to the vertical direction, while in the open state (or in the closed state), then the tips of the left and right thumbs will naturally contact the sensor units 1X.

It should be noted that the layout configurations of the sensor units 1X shown in FIG. 30 through FIG. 32 are nothing more than examples, and it is obvious that other various layout configurations are possible.

Although the management device 200 is not shown, it is provided with a storage function for storing data that is transmitted from the health management terminal 100, a management function for managing the transition in the state of health of an individual based on the data stored in the storage device 210, and a judging function for comparing the data transmitted from the health management terminal 100 with past data stored in the storage device 210, and for making judgments about the transition of the individual's state of health. In addition to data relating to medical information, the data that is transmitted from the health management terminal 100 may also include the service user's personal data. Data such as a health insurance number, consultation ticket, ID card or drug history may be used as the data that is transmitted from the health management terminal 100, and such personal information is stored in an unshown, personal information data base in the storage device 210.

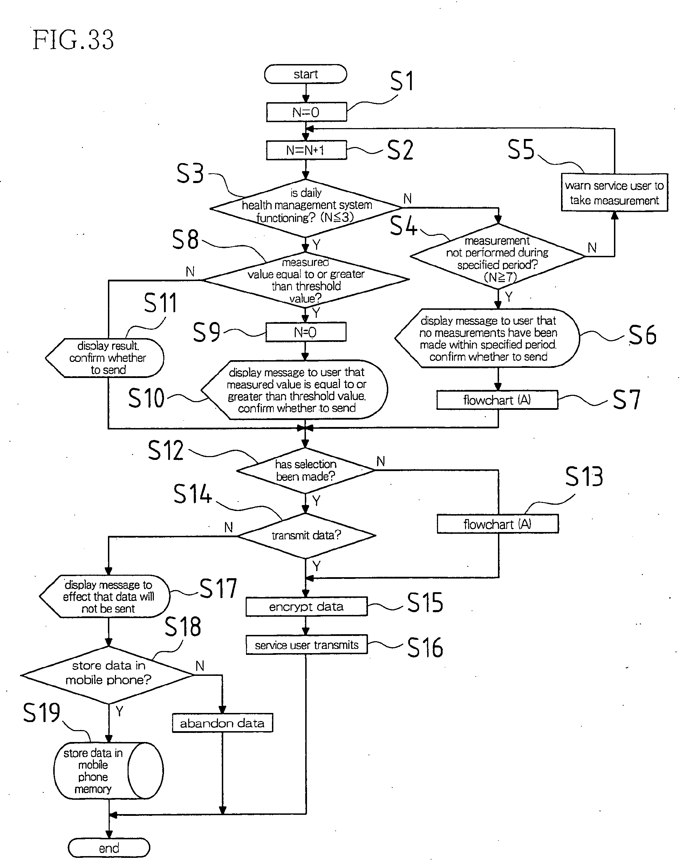

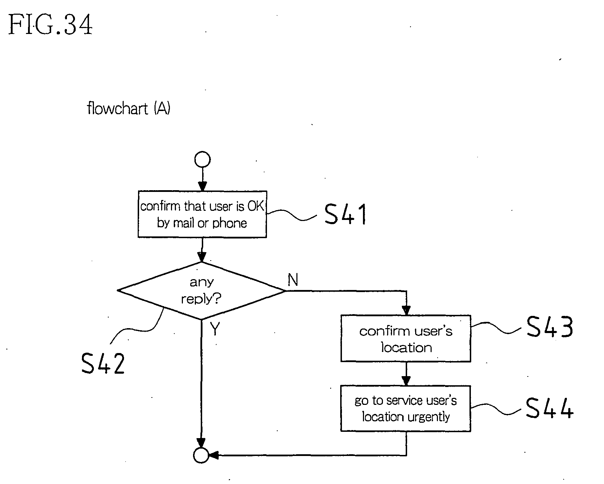

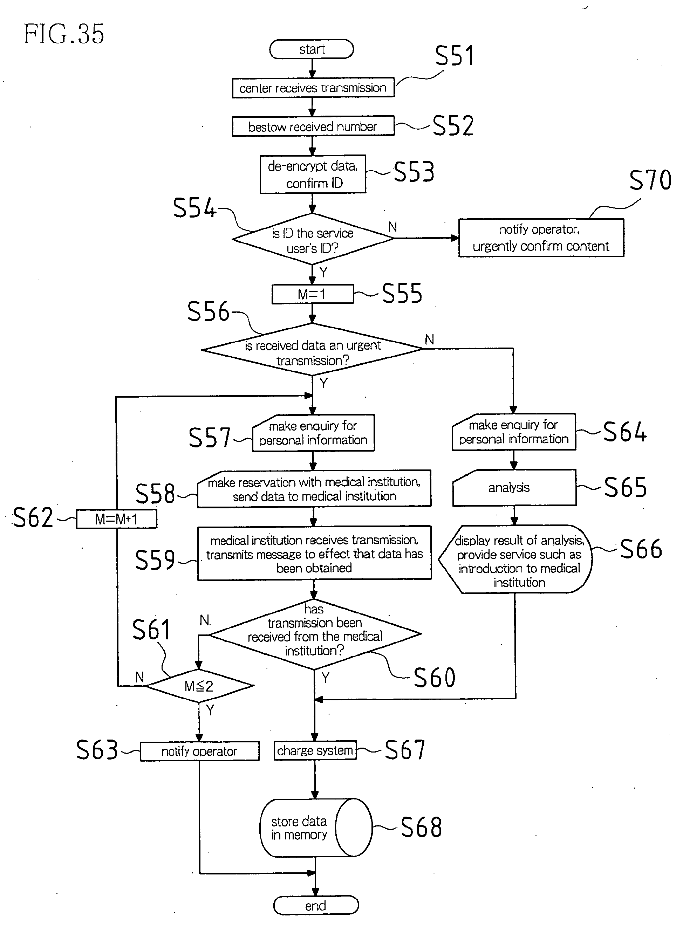

The operations for collecting and transmitting medical information with the health management terminal 100, and processing operations based on information received with the management device 200 in the health management system of the configuration described above, are described next with reference to the flowcharts shown in FIG. 33 to FIG. 35. FIG. 33 is a flowchart showing the processing operation of the health management terminal 100 and FIG. 34 and FIG. 35 are flowcharts showing the processing operations of the management device 200.

### Description of Processing Operations of a Mobile Phone serving as the Health Management Terminal 100

First, a count value N of an internal counter, not shown, is set to 0 (step S1). Next, the count value N is set to N+1 (step S2), after which a judgment is made, from the data stored in a storage device 305 in the mobile phone, as to whether or not medical information has been sensed by the sensor unit 1X of the medical information detection apparatus mounted on the mobile phone within a specified period (step S3 and step S4). As a result, if sensing has not been performed within a specified period (if the result is judged to be NO at step S4), then a screen that warns the service user to measure his medical information is displayed on the display screen 306 (corresponding to the display screen 101 provided on the external face of the side of the main unit that is displayed, shown in FIG. 31, for example) (step S5), after which the procedure returns to step S2, and +1 is added to the count value. Such a process is repeated until the procedure judges YES at step S3, or until the count value N of the measurements in the specified period reaches 7. If the specified period is counted seven times without medical information being sensed by the sensor unit 1X (if the result is judged to be YES at step S4), then after displaying a message on the display device 306 to warn the service user (in the mobile phone, the display device 101 provided on the outside face of the side 103 of the displayed main unit shown in FIG. 32, for example) to the effect that no measurements of medical information have been taken within the set period, the procedure confirms whether or not to transmit the warning to the management device 200 (step S6). If there is no response to the confirmation message from the service user, then a message to the effect that the service user did not confirm the warning is transmitted to the management device 200. In this case, the procedure moves to the process of step S7 (the process flow of flow chart (A) shown in FIG. 34).

That is to say, when the management device 200 receives information to the effect that the service user did not confirm the warning, the management device makes an enquiry by mail, for example, to the mobile phone requesting a reply within 24 hours, Or a direct phone enquiry is made immediately (step S41). If the service user replies, for example, and clear confirmation is made that there is no abnormality in the medical information (if the result is judged to be YES at step S42), then the procedure in the mobile phone returns to step S1, and the count value N of the internal counter is returned to 0. On the other hand, if there is no reply, or if it cannot be confirmed that the medical information is not abnormal (if the result is judged to be NO at step S42), then the location of the service user is confirmed using a positioning system attached to the mobile phone (step S43), and a response is taken, such as sending someone to the scene urgently (step S44) or making confirmation with a pre-registered emergency contact number. After this, the procedure on the mobile phone side returns to step S1, and the count value N of the internal counter is returned to 0.

On the other hand, if the medical information has been sensed by the sensor unit 1X within the specified period (if the result is judged to be YES at step S3), then the computing unit 303 of the medical information detection apparatus determines whether or not the results of measuring the medical information with the sensor unit 1X are equal to or greater than a threshold value that has been set in advance (step S8).

As a result, if the measured value is equal to or greater than the threshold value (if the result is judged to be YES at step S8), then the count value up to that point is returned to 0 (step S9), and the procedure confirms with the service user whether or not to transmit the data that is equal to or greater than the threshold value to the management device 200 at the management center (step S10). Since the individual medical information is private information with a high degree of confidentiality, it is necessary to obtain confirmation from the service user of the intention to transmit the information. Thus, the choice confirming the intention to transmit is given to the user.

On the other hand, if the measured value is less than the threshold value (if the result is judged to be NO at step S8), then the result of the measurement is displayed, and the procedure confirms with the user whether or not to transmit that information to the management device 200 at the health management center (step S11). Since the individual medical information is private information with a high degree of confidentiality, it is necessary to obtain confirmation from the service user of the intention to transmit the information. Thus, the choice confirming the intention to transmit is given to the user. However, in this case as well, if the selection is not made within a specified period, then the same process as that of step S7 is carried out.

If a specified time period passes without the user demonstrating a clear intent toward such an "intention to transmit" confirmation (if the result is judged to be NO at step S12), then since there is a possibility that the state of the service user's condition has deteriorated considerably, the procedure moves to the process in step S13 (the process flow in flowchart (A) shown in FIG. 34) in this case also.

That is to say, when the management device 200 receives information to the effect that the specific time period passed without the service user demonstrating his intent, the management device makes an enquiry by mail, for example, to that mobile phone, requesting a reply within 24 hours. Or a direct phone enquiry is made immediately (step S41). If the service user replies, for example, and clear confirmation is made that there is no abnormality in the medical information (if the result is judged to be YES at step S42), then the procedure in the mobile phone returns to step S1, and the count value N of the internal counter is returned to 0. On the other hand, if there is no reply, or if it cannot be confirmed that the medical information is not abnormal (if the result is judged to be NO at step S42), then the location of the service user is confirmed using a positioning system attached to the mobile phone (step S43), and a response is taken, such as urgently sending someone to the scene (step S44) or making confirmation with a pre-registered emergency contact number. After this, the procedure on the mobile phone side returns to step S1, and the count value N of the internal counter is returned to 0.

On the other hand, if the service user demonstrates his intent in step S12 (if the result is judged to be YES in step S12), then the procedure next confirms the intent to transmit the measured data (step S14). As a result, if the service user has both the intent to transmit, and selects "transmit" (if the result is judged to be YES in step S14), then the measured data is encrypted (step S15), and then transmitted to the management device 200 at the management center (step S16). Although it goes without saying that security on systems such as digital networks is high, the measured data is encrypted as a further precaution. However, if the network is a closed system with highly secure system structure, then it may not be necessary to encrypt the measured data.

If there is no intention to transmit the measured data (if the result in step S14 is NO), then a message is displayed to the effect that the data will not be transmitted (step S17), and the service user is made to choose whether or not to store information such as the measured data and the time and date, in the storage device 305 provided in the mobile phone (step S18). If the service user selects "store" (if the result of step S18 is judged to be YES), then the information such as the measured data and the time and date is stored in the storage device 305 in accordance with predetermined protocols (step S 19). On the other hand, if the service user chooses not to store the measured data (if the result of step S18 is judged to be NO) then the data is abandoned (step S20). This is so as not to store wrong data, such as medical information that has been erroneously collected from someone other than the service user, for use in data collation or health management, to be described later.

### Description of the Process Operations of the Managemen Device 200

When the management device 200 receives data from the mobile phone (step S51), a number is assigned to the data (step S52), and the data is decrypted and its ID confirmed (step S53). The received number is taken to be a number that is given uniquely, based on the date and time, for example. This is so that data can be extracted based on the received number, and so that the data processing does not take longer than a specified time when urgent data is received. Particularly, this measure is taken so that data required in an emergency is not overlooked.

After this, the received data is compared with personal data (the ID of the service user) stored in the storage device 210 to confirm whether or not it is the service user's data (step S54). As a result, if the data is confirmed to be the service user's data (if the result of step S54 is judged to be YES), then +1 is added to a count value M of an internal counter, not shown (step S55); and the received data is checked (step S56). On the other hand, if the received data could not be confirmed to be the service user's data (if the result of step S55 is judged to be NO) then the management device 200 quickly notifies the operator to confirm the content of the information (step S70). This measure is taken such that this data is not rejected for being data other than the service user's data due to some problem in the system, despite the fact that it is urgent information from the service user.

If, as a result of the check in step S56, the content of the received data is urgent (if the result of the step S56 is judged to be YES), then an enquiry is made to personal data that is stored in the storage device 210 (step S57), and arrangements are made to find, introduce, make a reservation, and send the data to a medical institution (step S58). Furthermore, that periodic data and personal data of the accumulated data, such as the service user's health insurance number, is transmitted to the terminal 300 of the medical institution (step S58). At this time, the data that is transmitted is encrypted and then transmitted. When the terminal 300 of the medical institution receives the personal data, a reply signal to the effect that the personal data has been obtained is transmitted to the management device 200 (step S59).

The management device 200 checks whether of not the reply signal has been received from the terminal 300 of the medical institution (step S60). As a result, is the reply signal has not been received despite transmitting the personal data (if the result of step S60 is judged to be NO), then the count value M of the internal counter is checked, and the procedure confirms that the count value M is less than two (that is to say, the data has been transmitted less than two times) (step S61). If the data has been transmitted two or fewer times (that is to say, it has been transmitted once) (if the result of step S61 is judged to be NO), then +1 is added to the count value of the internal counter (step S62) after which the procedure returns to step S57 and the personal data is transmitted again. On the other hand, if the personal data has been transmitted two or more times, (if the result of step S63 is judged to be YES), then the fact that the personal data could not be transmitted to the terminal 300 of the medical institution is notified to the operator (step S63).

As a result of the check in step S60, if the response signal is being received (if the result of step S60 is judged to be YES), then the necessary fee is charged to the service user (step S68) depending on the content of the service, and the necessary data is transferred to and stored in the storage device 210 (step S69).

On the other hand, if, as a result of the check in step S56, the received data has a low level of urgency (if the result of step S56 is judged to be NO), then an enquiry is made to the personal information in the storage device 210 (step S64), the data is extracted from the accumulated data, and after processing such as comparing and analyzing the data (step S65), the result of the analysis is transmitted to the service user's mobile phone and displayed to provide various services to the service user (step S66). The service content may include preparations such as recommending and making an appointment for a health check at the medical institution, reserving or introducing a fitness club, or proposing a menu for home delivery meals. If the service user then chooses the desired services from among the various services that are proposed, then the health management center can prepare an appointment for a health consultation at the medical institution, or make reservations or introductions to a health club using the management device 200. After this, the necessary fee is charged to the service user based on the service user's state of health and the content of the service (step S67), and the necessary data is transferred to the storage device 210 and stored (step S68).

The present invention can be embodied and practiced in other different forms without departing from the spirit and essential characteristics thereof. Therefore, the above-described embodiments are considered in all respects as illustrative and not restrictive. The scope of the invention is indicated by the appended claims rather than by the foregoing description. All variations and modifications falling within the equivalency range of the appended claims are intended to be embraced therein.

## Claims

1. A medical information detection apparatus, wherein movement of blood that is pumped from a heart is regarded as a pulse wave, the medical information detection apparatus comprising:
pulse wave detecting means constructed of a light emitting and receiving device, or a pressure-sensing element, detecting the pulse wave; and
impedance detecting means constructed of a current application electrode and a voltage detection electrode.

2. The medical information detection apparatus according to claim 1,
wherein a plurality of light receiving devices are mounted to constitute the light emitting and receiving device.

3. The medical information detection apparatus according to claim 1,
wherein a plurality of light emitting devices are mounted to constitute the light emitting and receiving device.

4. The medical information detection apparatus according to claim 1,
wherein a plurality of both light emitting devices and light receiving devices are mounted to constitute the light emitting and receiving device..

5. The medical information detection apparatus according to any one of claims 1 to 4,
wherein a wavelength of emitted light that is emitted by the light emitting device, and a wavelength of received light to which the light receiving device is sensitive, that constitute the light emitting and receiving device, are wavelengths that are in the near infrared light or infrared light region.

6. The medical information detection apparatus according to claim 5,
wherein the wavelength of the light emitted by the light emitting device, and the light sensitive wavelength of the light receiving device is in a wavelength band of 360 to 660 nm.

7. The medical information detection apparatus according to claim 5,
wherein the light receiving device is sealed in a resin that contains a component for cutting visible light.

8. The medical information detection apparatus according to claim 5,
wherein a visible-light cutting filter is disposed in front of a light receiving surface of the light receiving device.

9. The medical information detection apparatus according to claim 5,
wherein the light emitting device is sealed in a resin that contains a component for cutting visible light.

10. The medical information detection apparatus according to claim 5,
wherein a visible-light cutting filter is disposed in front of a light emitting unit of the light emitting device.

11. The medical information detection apparatus according to any one of claims 1 to 4,
wherein an IrDA device is mounted as the light emitting and receiving device of the pulse wave detecting means.

12. The medical information detection apparatus according to any one of claims 1 to 4,
wherein in the medical information detection apparatus, a protruding portion is provided on a peripheral portion of said pulse wave detecting means, such that a part of a person's body contacts the pulse wave detecting means with optimum pressure when detecting the pulse wave.

13. The medical information detection apparatus according any one of to 1 to 4,
wherein the pulse wave detecting means and the impedance detecting means are formed as a single piece so as to be able to detect the same location.

14. The medical information detection apparatus according to claim 13,
wherein the pulse wave detecting means and the impedance detecting means are disposed in at least two locations.

15. The medical information detection apparatus according to any one of claims 1 to 4, further comprising:
pulse wave propagation time calculating means for calculating the pulse wave propagation time based on signals detected by the pulse wave detecting means;
impedance calculating means for calculating impedance based on signals detected by the impedance detecting means; and
storing means for storing the calculated results.

16. The medical information detection apparatus according to any one of claims 1 to 4,
wherein the light emitting and receiving device is fixed to a holder, and the current application electrode and the voltage detection electrode are formed as a single piece with the holder.

17. The medical information detection apparatus according to any one of claims 1 to 4,
wherein resin is filled between the light emitting and receiving device and the current application electrode and the voltage detection electrode; and
wherein the light emitting and receiving device, and the current application electrode and the voltage detection electrode are formed as a single piece in the sealing resin.

18. The medical information detection apparatus according to any one of claims 1 to 4,
wherein a front peripheral portion of the light emitting and receiving device is sealed with an electrically conductive resin, and the current application electrode and the voltage detection electrode are formed by the electrically conductive resin used for sealing.

19. The medical information detection apparatus according to claim 1,
wherein a plurality of the pulse wave detecting means, a plurality of the impedance detecting means, blood pressure measuring means for receiving a pulse wave signal from the plurality of pulse wave detecting means and determining a blood pressure level from the time difference between the pulse waves detected at the two or more locations, and body fat percentage calculating means for calculating body fat percentage from impedance values between the plurality of impedance detecting means and physical data that is input in advance, are contained in a portable casing.

20. The medical information detection apparatus according to claim 19,
wherein the wavelength of the light emitted by the light emitting device, and the light sensitive wavelength of the light receiving device is in a wavelength band of 780 to 1000 nm.

21. The medical information detection apparatus according to claim 19,
wherein the wavelength of the light emitted by the light emitting device, and the light sensitive wavelength of the light receiving device is in a wavelength band of 360 to 660 nm.

22. The medical information detection apparatus according to any one of claims 19 to 21,
wherein one of the pulse wave detecting means and one of the impedance detecting means are formed as a single piece so as to sense a single location on a body.

23. The medical information detection apparatus according to any one of claims 19 to 21,
wherein the medical information detection apparatus is incorporated with a flip-type mobile phone as a single piece, and blood pressure and body fat percentage are measured while the mobile phone is in a closed state.

24. The medical information detection apparatus according to any one of claims 19 to 21,
wherein the medical information detection apparatus is incorporated with a flip-type mobile phone as a single piece, and blood pressure and body fat percentage are measured while the mobile phone is in an unfolded state.

25. The medical information detection apparatus according to any one of claims 19 to 21,
wherein the medical information detection apparatus is incorporated with a non-folding-type mobile phone as a single piece to measure blood pressure and body fat percentage.

26. The medical information detection apparatus according to any one of claims 19 to 21, further comprising:
a display unit for displaying the measured results of the blood pressure level and the body fat percentage.

27. The medical information detection apparatus according to any one of claims 19 to 21,
wherein an IrDAdevice and a light receiving element are used as the pair of the light emitting element and the light receiving element.

28. The medical information detection apparatus according to claim 27,
wherein the medical information detection apparatus is incorporated with a flip-type mobile phone as a single piece, and blood pressure and body fat percentage are measured while the mobile phone is in a closed state.

29. The medical information detection apparatus according to claim 27,
wherein the medical information detection apparatus is incorporated with a flip-type mobile phone as a single piece, and blood pressure and body fat percentage are measured while the mobile phone is in an unfolded state.

30. The medical information detection apparatus according to claim 27,
wherein the medical information detection apparatus is incorporated with a non-folding-type mobile phone as a single piece to measure blood pressure and body fat percentage.

31. The medical information detection apparatus according to any one of claims 19 to 21,
wherein the electrodes of the impedance detecting means can also be used as electrodes of a sensor for recognizing fingerprints.

32. The medical information detection apparatus according to any one of claims 19 to 21,
wherein the medical information detection apparatus is configured to be capable of transmitting information of the blood pressure level and the body fat percentage, which are the measured results to an external device, or of obtaining physical data of the subject from the external device by an IrDA device.

33. The medical information detection apparatus according to any one of claims 19 to 21, further comprising:
a blood sugar level sensor.

34. The medical information detection apparatus according to claim 33,
wherein the blood sugar level sensor is configured to measure the blood sugar level by a chemical detection method.

35. The medical information detection apparatus according to claim 33,
wherein the blood sugar level sensor is configured to measure the blood sugar level by an optical detection method.

36. The medical information detection apparatus according to any one of claims 19 to 21,
wherein the medical information detection apparatus is configured to transmit the measured medical information to a medical institution, for example by a communication function such as the internet, and after a doctor has diagnosed the medical information, to receive the results of the diagnosis using the communication function, such as the internet.

37. The medical information detection apparatus according to any one of claims 19 to 21,
wherein the medical information detection apparatus is configured to execute a piece of application software by an application software executing function, based on the measured medical information.

38. The medical information detection apparatus according to any one of claims 19 to 21, further comprising:
an alarm function for notifying a time to measure blood pressure and body fat.

39. The medical information detection apparatus according to any one of claims 19 to 21,
wherein measurement of the medical information is incorporated into a personal computer.

40. The medical information detection apparatus according to any one of claims 19 to 21,
wherein measurement of the medical information is incorporated into a mouse for using personal computer.

41. The medical information detection apparatus according to claim 1,
wherein a plurality of the pulse wave detecting means, a plurality of the impedance detecting means, blood pressure measuring means for receiving a pulse wave signal from the plurality of pulse wave detecting means and determining a blood pressure level from the time difference between the pulse waves detected at the two or more locations, and body fat percentage calculating means for calculating body fat percentage from impedance values between the plurality of impedance detecting means and physical data that is input in advance, are incorporated in an interior component of a vehicle.

42. The medical information detection apparatus according to claim 41,
wherein the interior component of the vehicle is a steering wheel.

43. The medical information detection apparatus according to claim 41,
wherein the interior component of the vehicle is a shift lever.

44. The medical information detection apparatus according to claim 41,
wherein the interior component of the vehicle is a seat.

45. The medical information detection apparatus according to any one of claims 41 to 44,
wherein the near-infrared region, which is 780 to 1000 nm, is used as the wavelength of the light emitted by the light emitting device, and as the light sensitive wavelength of the light receiving device.

46. The medical information detection apparatus according to any one of claims 41 to 44,
wherein light of a wavelength of 360 to 660 nm is used as the wavelength of the light emitted by the light emitting device and the light sensitive wavelength of the light receiving device.

47. The medical information detection apparatus according to any one of claims 41 to 44,
wherein one of the pulse wave detecting means and one of the impedance detecting means are formed as a single piece so as to sense a single location on a body.

48. The medical information detection apparatus according to any one of claims 41 to 44,
wherein an IrDA device and a light receiving element are used as the pair of the light emitting element and the light receiving element.

49. The medical information detection apparatus according to any one of claims 41 to 44,
wherein the medical information detection apparatus is configured to be capable of transmitting information of the blood pressure level and the body fat percentage, which are the measured results, to an external device by the IrDA device, or of obtaining physical data of the subject from the external device.

50. The medical information detection apparatus according to any one of claims 41 to 44, further comprising:
a blood sugar level sensor.

51. The medical information detection apparatus according to claim 50,
wherein the blood sugar level sensor is constituted to measure the blood sugar level by a chemical detection method.

52. The medical information detection apparatus according to claim 50,
wherein the blood sugar level sensor is constituted to measure the blood sugar level by an optical detection method.

53. The medical information detection apparatus according to any one of claims 41 to 44, further comprising:
notifying means for judging whether or not the subject is in a condition in which they are capable of driving the vehicle, based on the measured values, and for notifying the result of that judgment,

54. The medical information detection apparatus according to any one of claims 41 to 44, further comprising:
air-conditioner adjusting means for adjusting a set air strength and a set temperature of an air-conditioning apparatus of the vehicle, based on the measured results.

55. The medical information detection apparatus according to any one of claims 41 to 44, further comprising:
acoustic controlling means for adjusting a volume of an acoustic device in the vehicle, based on the measured results.

56. The medical information detection apparatus according to claim 1,
wherein a sensor comprising the pulse wave detecting means and the impedance detecting means is contained in any structural part of a robot that has a numerical input function, a display function, a transmission and receiving function, a storage function, an alarm function, an application software execution function, a calculation function and a control function.

57. The medical information detection apparatus according to claim 56,
wherein the sensor is provided on a part of the robot that touches a living organism.

58. The medical information detection apparatus according to claim 56 or claim 57,
wherein the medical information sensed by the sensor is at least one or more of an electrocardiac signal, blood pressure, pulse rate, pulse wave, body temperature, body fat, bone density, blood oxygen concentration and blood sugar level.

59. The medical information detection apparatus according to claim 58,
wherein the sensor is constructed of a combination of the light emitting device and the light receiving device; and
wherein the pulse rate is measured by sensing with the sensor, utilizing the light absorbing characteristics of oxy-hemoglobin and hemoglobin.

60. The medical information detection apparatus according to claim 58,
wherein the sensor is constructed of a pressure sensitive element; and
wherein the pulse rate is measured by sensing with the pressure sensitive element.

61. The medical information detection apparatus according to claim 58,
wherein the blood pressure is determined by calculating the time difference between an electrocardiac signal and a pulse wave.

62. The medical information detection apparatus according to claim 58,
wherein the blood pressure is determined by a difference in pulse wave propagation speed between two measurement regions on a body.

63. The medical information detection apparatus according to claim 61 or claim 62,
wherein the pulse wave is measured by sensing with a sensor using the sensor constructed of a combination of the light emitting device and the light receiving device, and utilizing the light absorbing characteristics of oxy-hemoglobin and hemoglobin.

64. The medical information detection apparatus according to claim 61 or claim 62,
wherein the pulse wave is measured by measuring pressure fluctuations with a pressure sensitive element.

65. The medical information detection apparatus according to claim 58,
wherein the sensor is constructed of a combination of the light emitting device and the light receiving device, and the blood oxygen concentration is determined with the sensor by using light of a plurality of wavelengths to measure the degree of light absorption of oxy-hemoglobin and hemoglobin in the blood.

66. The medical information detection apparatus according to claim 58,
wherein the body fat or the bone density is determined by passing a current between two measurement regions, and measuring the impedance of a living organism by the voltage between those two points.

67. The medical information detection apparatus according to claim 58,
wherein the electrocardiac signal is determined by measuring between two regions on a body using an inductive method.

68. The medical information detection apparatus according to claim 58,
wherein the sensor includes a pyroelectric sensor, and the body temperature is determined by measuring inside the ear with the pyroelectric sensor.

69. The medical information detection apparatus according to claim 58,
wherein the sensor includes a plurality of near infrared light sources and light receiving elements, and the blood sugar level is measured by the sensor using a non-invasive method.

70. The medical information detection apparatus according to claim 56 or claim 57, further comprising:
a network connection function.

71. The medical information detection apparatus according to claim 70,
wherein information on another device can be obtained by utilizing the network connection function.

72. A health management system, comprising:
a portable-type health management terminal provided with a communication function, and a management device in a health management center that can be connected via a communication network;
wherein the health management terminal comprises:
at least either one of sensing means constructed of a medical information detection apparatus according to any one of claims 1 to 71 that can sense medical information; or inputting means into which a health state can be input;
judging means for judging the health state based on the information obtained by these means;
storing means for storing the information; and
transmitting means for transmitting the information and the information of the judgment result via the communication network; and
wherein the management device comprises:
storing means for storing, in a storage device, the information that is transmitted; and
managing means for managing the transition of an individual's health state based on the information stored in the storage device.

73. The health management system according to claim 72,
wherein the health management terminal is a mobile phone; and
wherein the sensing means is disposed in the vicinity of a location to which a finger will naturally come when the mobile phone is being used.

74. The health management system according to claim 72 or claim 73,
wherein the information that is sensed by the sensing means is a pulse wave, and a living organism's impedance.

75. The health management system according to claim 72,
wherein a terminal of a facility that can provide a necessary service to a user holding the health management terminal is connected to the communication network, and the management device communicates information relating to the provision of the service to the terminal of the facility.

76. The health management system according to claim 72,
wherein the medical information collected by the health management terminal is at least any one of body temperature, blood pressure, pulse rate, pulse wave, heart rate, body weight, body fat, amount of internal organ fat, bone density, skin moisture, skin oil, blood oxygen concentration, blood sugar level, blood composition, cholesterol level, uric acid level, brain waves, stool amount and stool composition.

77. The health management system according to claim 72,
wherein the management device further comprises:
judging means for comparatively calculating the data that is transmitted from the health management terminal with the past data stored on the storage device to judge the transition in the state of health of the user.

78. The health management system according to claim 72,
wherein the health management terminal transmits data including information such as health insurance number, consultation ticket, ID card or drug history, stored by the storing means.
